# EUROPEAN PATENT APPLICATION

(11) **EP 4 527 380 A1**
(43) Date of publication of application: **26.03.2025**
(21) Application number: 23198227.3
(22) Date of filing: 19.09.2023
(51) Int. Cl.: A61K 9/51, A61K 9/127

(54) **LIPID NANOPARTICLES**

(71) Applicant: Thermosome GmbH, 82152 Planegg (DE)
(72) Inventor: Petrini, Matteo, 82152 Planegg (DE); Mayer, Susanne, 82152 Planegg (DE); Petrov, Orlin, 82152 Planegg (DE); Michaelis, Uwe, 82152 Planegg (DE); Massing, Ulrich, 82152 Planegg (DE); Lindner, Lars, 81377 München (DE)
(74) Representative: Weickmann & Weickmann PartmbB

(57) **Abstract**

The present invention relates to compositions, and in particular to lipid nanoparticles, comprising at least one oligoglycerol-containing lipid. The compositions are in particular suitable for delivery of RNA.

## Description

The present invention relates to compositions, and in particular to lipid nanoparticles (LNP), comprising at least one oligoglycerol-containing lipid. The compositions are in particular suitable for delivery of RNA.

In the last years RNA based therapeutics have emerged as a promising new drug class. The effectiveness of RNA based vaccine has already been impressively demonstrated in the COVID-19 pandemic. Various types of RNA molecules such as anti-sense oligonucleotides, small interfering RNA (siRNA) & microRNAs (mi-RNAs), crRNA, tracrRNA or sgRNA could facilitate targeting mRNAs or DNA using Watson-Crick base-pairing strategy (Zhu, et al. 2022). Beside the use of messenger RNA (mRNA) as prophylactic vaccine against infectious diseases or therapeutic vaccine for cancer treatment, RNA can be used as therapeutic agent for treatment of chronic or genetic diseases (Li, et al. 2022).

For parenteral applications "naked" mRNA needs to be protected against degradation. This applies in particular to systemic administrations, e.g., intravenous, intraarterial, intramuscular and subcutaneous injections or infusions of mRNA as well for nasal application or administration of mRNA by breathing into the lungs - usually through the mouth by inhalation or nose by nebulization or spraying. Further the mRNA needs to be entrapped into a so-called non-viral delivery system to make it transfection competent, i.e., enabling the binding of mRNA to the surface of a target cell of interest, followed by uptake into the cell via endocytosis and release from the endosome into the cytosol, where protein expression takes place.

To overcome the transfection and degradation problems with "naked" mRNA, polymer- and in particular lipid nanoparticle (LNP) based non-viral delivery systems have been developed in the last decades. Currently, all approved mRNA COVID-19 vaccines and most RNA drug development programs in oncology as well as non-oncology diseases are utilizing LNP technology to facilitate mRNA, siRNA, crRNA, tracrRNA or sgRNA delivery into the cytoplasm of target cells.

Current LNP technology for delivery of mRNA, siRNA or RNA species for CR!SPR-Cas9 mediated genome editing uses four main structural components to build a lipid nanoparticle: a neutral or zwitterionic so-called "helper" phospholipid, cholesterol, a polyethylene-glycol (PEG)-lipid, and an ionizable lipid, which is, depending on the pH value, neutral or cationic. The ionizable, pH-sensitive, lipid contains one or more positively charged ionizable amine groups (at low pH) and has been optimized for encapsulation of the anionic RNA, as for example mRNA, siRNA, crRNA, tracrRNA or sgRNA (i.e., particle formation) and cellular uptake of the LNP (internalization by endocytosis). Further the ionizable lipid is necessary and mainly responsible for effective intracellular delivery by facilitating membrane fusion during internalization (endocytosis) and RNA release from the endosome into the cytosol, the so-called endosomal escape (Whitehead, et al. 2014); (Edo Kon 2022); (Sabnis, et al. 2018). The phospholipid 1,2-distearoly-*sn*-glycero-3-phosphocholine (DSPC) supports the cationizable lipid in cellular uptake and cellular trafficking of the LNP and the encapsulated RNA (Arteta, et al. 2018). The PEG-lipids have been engineered i) to regulate the LNP size during manufacturing and act as a steric barrier to prevent aggregation during storage as well as ii) to prevent unspecific interaction with cellular components after systemic administration.

Examples for typical lipid nanoparticles (LNPs) are the mRNA based LNPs *Comirnaty* (BioNTech/Pfizer's COVID-19 vaccine (Formulation B)), *Spikevax* (Moderna's COVID-19 vaccine (Formulation A)) and *Onpattro* (Alnylam, first interfering RNA (RNAi) drug approved by the FDA). They contain different ionizable cationic lipids, the zwitterionic DSPC as helper lipid, cholesterol, and PEGylated lipids.

The exact architecture and structure of LNPs and their encapsulated RNA molecules is subject of an intense debate. Nevertheless, currently most accepted models postulate that most if not all PEG lipids and the greater part of the DSPC helper lipids are located at the surface of the LNPs. Further, in contrast to a classical unilamellar liposome, the LNP contains lipids in its interior. During the manufacturing process, which involves a pH shift from acidic to neutral condition, the bulk of the ionizable lipid segregates into a central oil phase. The exact localization of the RNA inside the core differs between the small siRNA and the up to 100-fold larger mRNA. It is also common understanding that the LNP architecture might also be influenced by the nature of the mRNA or siRNA, their molecular weights, their sequence and chemical composition of the nucleotides (for review see e.g.: (Schoenmaker, et al. 2021)).

The chemical structure of the lipids (ionizable, helper and PEG) and the composition of the formulation, i.e., the molar ratios of the different components, mainly determines the architecture and surface charge of the LNPs carrying mRNA or siRNA. PEG lipids play an important role for the size of the final LNP. For example, it was shown (Arteta, et al. 2018) that by changing the relative amount of PEG lipid from 3 to 0.25 mol% the size of the final mRNA carrying LNP were increased from 45 nm to 135 nm (number average diameter). A smaller relative proportion of PEG lipids enables lower surface-to-volume ratios and consequently higher particle sizes. Most important for the *in vivo* (and *in vitro*) performance of the mRNA carrying LNP is the nature of the ionizable lipid, the chemical structure of the ionizable head group and the lipid tails, as there are for example the presence of branched or linear lipid tails, the position of ester bonds and the distance between the head group and the other motifs (Whitehead, et al. 2014); (Sabnis, et al. 2018).

Further, the manufacturing procedure for the RNA-LNPs beside the nature of the RNA itself (e.g., siRNA versus mRNA, see above) has an impact on the final structure of the mature RNA-LNP. The structure of the RNA-LNP, its size, charge, and composition, in turn are the major determinants for its biodistribution (organ, tissue and/or target cell specificity) and the potency of the entrapped RNA - as measured by level and duration of protein expression in case of mRNA, gene-silencing in case of siRNA or DNA editing efficiency in case of the CRISPR/Cas based approach.

It is important to note that for systemic administration each target cell type and route of administration requires specific evaluation and optimization of all these parameters. Thus, there is a high need to significantly expand the repertoire of LNP formulations and components to adapt the delivery of the therapeutic RNA for a specific disease treatment.

Currently used COVID-19 mRNA vaccines are composed of LNPs incorporating PEGylated lipids into their surface, thereby determining particle size during manufacturing, and preventing aggregation during storage and in tissues after IM (intramuscular), or SC (subcutaneous) administration. These vaccines contain diffusible PEG lipids with relatively short lipid chains (C14) which dissociate from the LNP after administration thereby generating transfection competent LNPs (for review see, e.g., (Kulkarni, Witzigmann und Chen 2019)). The COVID-19 mRNA vaccines can be considered as overwhelmingly safe. Most recent reports describe similar rates of allergic reactions and anaphylaxis to mRNA COVID-19 vaccines as to other vaccines (Jaggers and Wolfson 2023). By April 2022, the reported estimated rate for anaphylaxis has been between 2.5 and 4.7 per million (Jaggers and Wolfson 2023); (Tenchov, Sasso und Zhou 2023). Some of the symptoms in recipients of the COVID-19 mRNA vaccines are similar to that in patients after systematically administered Doxil, which is a PEGylated liposome carrying the cytotoxic drug doxorubicin. However, the frequency of these reactions to the mRNA vaccines has been much lower than that to Doxil (Szebeni, et al. 2022).

The polyethylene glycol (PEG)-lipids are considered as candidate allergens to cause the rare anaphylactic reactions against the mRNA COVID-19 vaccines (e.g., Banerji, 2021), although it is reported that some patients with known PEG allergy can tolerate the mRNA vaccine (Picard, et al. 2022); (Brockow, et al. 2022).

Although hypersensitivity is a rare adverse event it can be fatal. Accordingly, there is still a need for improved mRNA vaccines where the PEG-lipid is omitted in the LNP without diminishing the performance of the vaccines. Considering the exceptional importance of the mRNA vaccines technology for the fight against emerging new viral diseases underlines the necessity to avoid undesirable immune reactions and to further improve mRNA vaccines.

In contrast to the intramuscular injected COVID-19 mRNA vaccines, the use of RNA-based drugs for therapeutic treatment of many diseases will require systemic administration of the LNPs into the blood circulation. Incorporation of diffusible PEG-lipids into the LNPs confer a half-life of 15 min in circulation which is too short to allow extra-hepatic organ targeting (Kulkarni, Witzigmann und Chen 2019). Persistent PEG-lipids with longer lipid chains (e.g., C18) and increased amount could present a possible optimization route to diminish the clearance of LNPs from blood. Further, the size, composition, and charge of the LNPs need to be adapted for *in vivo* applications, thus improving tissue targeting and efficiency of encapsulated RNA. However, it has been reported that PEGylated nanocarriers do not necessarily escape the detection of the immune system. Unexpected immune reactions against PEGylated nanocarriers, mainly the formation of anti-PEG antibodies after initial injection, might lead to accelerated blood clearance (ABC), i.e., the increased clearance and reduced efficacy of PEGylated nanocarriers following repeated administrations (see for a comprehensive review: (Tenchov, Sasso und Zhou 2023)). Also, the possible presence of pre-existing antibodies in the absence of treatment with a PEGylated nanocarrier is known (Yang und Lai 2015), although the relevance is discussed controversially. The presence of anti-PEG antibodies could also play a role in the occurrence of hypersensitivity reactions like complement activation related pseudoallergy (CARPA). Most of the data have been reported with PEGylated classical nanocarriers (e.g., liposomes) and PEGylated proteins. It is debatable to which extend these observations will also apply for the systemic administration of RNA carrying LNPs. Nevertheless, for repeated administrations of therapeutic RNA LNP drugs, the immunosafety of the RNA carrying LNPs is of exceptional importance. Especially for the sometimes-lifelong treatment of chronic diseases adverse immune reactions must be prevented.

Unshielded liposomes or LNPs encapsulating RNA possess poor physicochemical characteristics for systemic delivery. Their surface, especially in case of cationic LNPs or liposomes, leads to nonspecific interaction with (mostly anionic) blood components and uptake by macrophages, resulting in rapid clearance by the reticuloendothelial system (RES). Consequently, after administration of unshielded LNPs into the circulation by injection or infusion predominantly so-called first-pass organs are targeted ("first-pass" organs).

One solution of the problem is the use of hydrophilic polymers attached to the particle's surface. Most commonly PEG has been used, although other polymers have been described (Heyes, et al. 2006), (Nogueira, et al. 2020).

Further, it is reported that the presence of hydrophilic polymers with a large head group is a prerequisite for the manufacturing of RNA-LNPs with well-defined size suitable for the parenteral administration. RNA-LNPs do not represent highly structured particles, but due to the physicochemical properties of their components they are largely self-assembling systems.

The established standard manufacturing processes use a low pH environment for the generation of a heterogeneous population of small LNPs, whereby the positively charged ionizable lipids interact with the negatively charged mRNA or siRNA. Induced by a pH shift to neutral value a reorganization of the different lipids and the RNA occurs. During this process the ionizable lipids diffuse into the center of the LNP particles and the PEG-lipid as well as the helper lipids assemble at the surface of the particle. Accordingly, after this maturation process, different LNP compartments can be detected.

In the presence of PEG-lipids a defined mRNA-LNP size is achieved when the concentration of PEG-lipid in the outer monolayer is sufficiently high to inhibit further fusion of the smaller LNPs. In the absence of PEG-lipid the LNPs continuously fuse during the manufacturing process to large particles with a heterogeneous size distribution which do not allow any more *in vivo* administration (Kulkarni, Witzigmann und Chen 2019); (Schoenmaker, et al. 2021). However, it has been shown that the presence of PEG does affect the intracellular delivery and trafficking of non-viral nucleic acid delivery particles. PEG on the surface of RNA carrying LNPs prevents fusion of LNPs with cell membranes thus decreasing cellular uptake and endosomal escape processes. This results into low gene expression (mRNA) or gene silencing potential (siRNA) (Woodle und Lasic 1992); (Heyes, et al. 2006).

A further disadvantage of the PEGylated mRNA carrying LNPs is that they potentially induce an antibody response against the PEG moiety. This could negatively impact their potential for therapies where repeated administration of PEGylated LNPs would be necessary. This is especially true for lifelong treatment of non-oncology diseases, e.g., protein replacement or substitution therapies in genetically inherited diseases.

Alternative hydrophilic headgroups which have been described still exhibit these disadvantages. Representative for this class of alternative shielding agents are LNPs functionalized with polysarcosine (pSar). Depending on the molar fraction of pSar lipid at the LNP surface, the LNPs showed one to several orders of magnitude lower transfection *in vitro* when compared to LNPs containing PEG-DMG, a PEG lipid which is used as shielding agent in the COVID-19 vaccine developed by Moderna Inc. (Nogueira, et al. 2020).

To overcome the limitations of these established or proposed alternative shielding agents several approaches have been followed.

One research approach tries to make the insertion and exposure of PEG lipids at the surface of the LNP transient. In principle this could be achieved by diffusible PEG-lipids that diffuse from the particle surface at a rate determined by the size of their lipid anchors (Ambegia, et al. 2005); (Hattori, et al. 2020). Other research approaches investigate sheddable PEG-lipids that possess a biodegradable bond between the polymer and the lipid anchor that is enzymatically cleaved in the extracellular environment after binding to the target tissue, for example a tumor or damaged tissue in cardiovascular diseases. A strong limitation of this concept is that the bulky headgroup of PEG also inhibits access to the enzymes which would be responsible for the linker cleavage. Alternatively, by introduction of a chemical bond which is sensitive to low pH, cleavage can occur intracellularly in the acidic endosomal compartment. The disadvantage of the latter approach is that the bulky PEG headgroup still negatively interferes with the uptake into the target cell. So, a strategy using diffusible PEG-lipids may be advantageous, rather than those where PEG is permanently bound or not cleaved until entry to the endosome.

Ambegia et al. systematically investigated PEG-lipids with different lengths of the lipid anchor (Ambegia, et al. 2005). Pharmacokinetic analysis of lipid nanoparticles containing complexed plasmid DNA confirmed a clear correlation between the stability of the PEG-lipids and circulation lifetime of the LNPs. The authors could also clearly show that the particles became more fusogenic over time as the PEG-lipid dissociated from the particle. This was due to the increased exposure of the fusogenic lipid bilayer as the outer PEG coating was removed. The speed of loss was a function of the length of the lipid anchor. The LNPs containing PEG-lipids with longer lipid anchors showed a slower dissociation from the particles and a reduced fusion behavior.

Since the dissociation of the PEG is necessary for a nucleic acid containing particle to become transfection competent, gene expression is related entirely to the rate at which the PEG-moiety is eliminated from the particle's bilayer. Consequently, the *in vitro* transfection efficiency of the investigated lipid nanoparticles containing complex plasmid DNA was highest with the PEG-lipid containing the shortest lipid anchor.

In this context it is important to mention that PEG ceramides containing only a very short lipid anchor (C8) could sufficiently increase the circulation time of plasmid DNA: lipid complexes compared to uncoated or 'naked' particles, thereby allowing targeting and gene expression at tumor sites (Tam, et al. 2000).

Summarizing, there is still a strong need for a shielding agent which moderately prolongs half-life of the LNPs in circulation, thereby allowing to bypass the "first-pass" organs. Further the shielding agent must not negatively interfere with binding of the LNPs to their target cells, i.e., it must allow efficient uptake into the target cells via endocytosis and must allow efficient release of the RNA from the endosome into the cytoplasm, mediated by the ionizable, pH-sensitive lipids. Such a novel shielding agent also has to maintain dispersion stability of the LNPs (e.g., no fusion or aggregation during storage and/or handling). Such a substitute for the currently used PEG lipids has not been described yet.

There is also a strong need to expand the repertoire of helper lipids for the future design of safe and efficacious LNPs. The so-called helper lipids strongly influence the properties of the LNPs, such as particle stability, delivery efficacy, tolerability and biodistribution (for review see, e.g., (Hou, et al. 2021)). Especially helper lipids such as DSPC and cholesterol also play an essential role by participating in the formation of LNPs. For example, DSPC, a zwitterionic phosphatidylcholine with two saturated lipid chains, stabilizes the structure of lipid nanoparticles, whereas 1,2-dioleoyl-sn-glycero-3-phosphoethanolamine (DOPE), which is a phosphatidylethanolamine with two unsaturated lipid chains, destabilizes endosomal membranes and facilitates endosomal escape of lipid nanoparticles (Hou, et al. 2021). The biodistribution and structure-function relationship of intravenously administered LNPs was systematically investigated by (Zhang, et al. 2021). By a high-throughput *in vivo* screening of a LNP library, the group studied how helper lipids influence the protein adsorption and the biodistribution of LNPs. Interestingly LNPs containing the helper lipid DOPE preferentially accumulated in the liver, while LNPs containing DOPC but were otherwise identical preferentially accumulated in the spleen.

Arteta et al. did a systematic investigation of the relationship between mRNA carrying LNP size, LNP architecture and protein expression after *in vitro* transfection (Arteta, et al. 2018). Focus of their study was the lipid composition at the surface of the LNP and the architecture of the LNP interior. Surprisingly, the phospholipid DSPC was mainly located at the surface of mRNA-LNPs and strongly influenced the transfection efficacy. Their data clearly showed that both surface composition and size of LNPs determine transfection efficacy, demonstrated in two different cell types *in vitro.* The effect of DSPC on protein expression was also remarkable, although DSPC promotes lamellar phase bilayers, thereby increasing the bilayer stability and should not be able to promote endosomal escape.

These investigations clearly show the striking effects of helper lipids on the biological efficacy of the LNPs. Helper lipids can play a central role in modulating the biodistribution, target specificity and protein expression of mRNA encapsulated in LNPs. This clearly shows the high need for a larger repertoire of innovative helper lipids for the future design of LNPs for systemic administration. This is especially true for the future development of LNPs which are designed without conjugation of a tissue specific targeting moiety as for example an antibody. This class of LNPs should preferentially target and specifically accumulate in a tissue or organ after systemic administration. Their inherent affinity to a specific tissue or organ will be determined by their lipid composition, including the helper lipid.

Thus, there is a great need for lipids which are in particular suitable as excipients for improved drug delivery systems such as improved LNPs.

The present invention relates to a composition comprising:
a) at least one oligoglycerol-containing lipid of Formula (I), 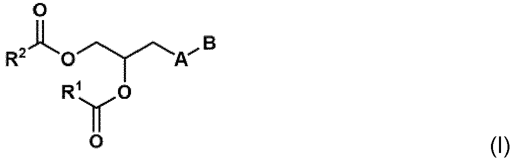
   wherein A = O-P(-O-)(=O)-O; or NR³ ; or N⁺R⁴R⁵ ;
   R¹ and R² each independently are a hydrocarbon group having 12 to 24 C-atoms;
   R³, R⁴ and R⁵ each independently are hydrogen or a hydrocarbon group having 1-5 C-atoms; and
   B = a linear or branched oligoglycerol group having from 2 to 20 glycerol units; and
b) at least one further lipid selected from
   (i) at least one cationic or cationizable lipid;
   (ii) at least one structural lipid;
   (iii) at least one helper lipid; and/or
   (iv) at least one PEG lipid.

In a preferred embodiment the present invention relates to a composition comprising:
a) at least one oligoglycerol-containing lipid of Formula (I), 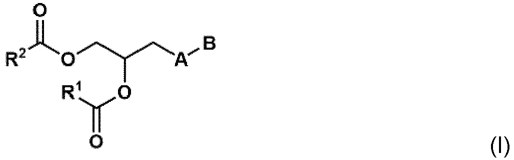
   wherein A = O-P(-O-)(=O)-O; or NR³ ; or N⁺R⁴R⁵ ;
   R¹ and R² each independently are a hydrocarbon group having 12 to 24 C-atoms;
   R³, R⁴ and R⁵ each independently are hydrogen or a hydrocarbon group having 1-5 C-atoms; and
   B = a linear or branched oligoglycerol group having from 2 to 20 glycerol units; and
b)(i) at least one cationic or cationizable lipid; and
b)(ii) at least one structural lipid.

In this embodiment, the at least oligoglycerol-containing lipid of Formula (I) may be comprised in the composition in an amount of about 1 mol% to about 25 mol%, preferably about 1.5 mol% to about 12 mol%, more preferably about 1.5 mol% to about 10 mol%;
the least one cationic or cationizable lipid may be comprised in an amount of about 40 mol% to about 55 mol%, preferably about 44 mol% to about 52 mol%, more preferably about 46 mol% to about 50 mol%;
and the at least one structural lipid in an amount of about 28 mol% to about 52 mol%, preferably about 32 mol% to about 46 mol%, more preferably about 34 mol% to about 44 mol%, based on the total lipid content of the composition.

In a further preferred embodiment the present invention relates to a composition comprising:
a) at least one oligoglycerol-containing lipid of Formula (I), 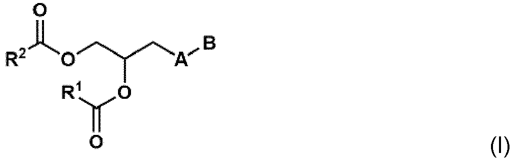
   wherein A = O-P(-O-)(=O)-O; or NR³ ; or N⁺R⁴R⁵ ;
   R¹ and R² each independently are a hydrocarbon group having 12 to 24 C-atoms;
   R³, R⁴ and R⁵ each independently are hydrogen or a hydrocarbon group having 1-5 C-atoms; and
   B = a linear or branched oligoglycerol group having from 2 to 20 glycerol units; and
b)(i) at least one cationic or cationizable lipid;
b)(ii) at least one structural lipid; and
b)(iii) at least one helper lipid.

In this embodiment, the at least oligoglycerol-containing lipid of Formula (I) may be comprised in the composition in an amount of about 1 mol% to about 25 mol%, preferably about 1.5 mol% to about 12 mol%, more preferably about 1.5 mol% to about 10 mol%;
the least one cationic or cationizable lipid may be comprised in an amount of about 40 mol% to about 55 mol%, preferably about 44 mol% to about 52 mol%, more preferably about 46 mol% to about 50 mol%;
the at least one structural lipid in an amount of about 28 mol% to about 52 mol%, preferably about 32 mol% to about 46 mol%, more preferably about 34 mol% to about 44 mol%;
and the at least one helper lipid in an amount of about 5 mol% to about 15 mol%, preferably about 7 mol% to about 12 mol%, more preferably about 9 mol% to about 11 mol%,based on the total lipid content of the composition.

In a further preferred embodiment, the present invention relates to a composition comprising:
a) at least one oligoglycerol-containing lipid of Formula (I), 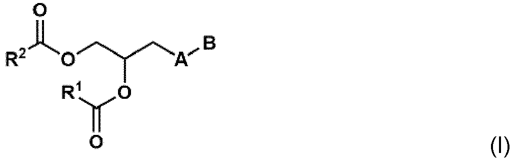
   wherein A = O-P(-O-)(=O)-O; or NR³ ; or N⁺R⁴R⁵ ;
   R¹ and R² each independently are a hydrocarbon group having 12 to 24 C-atoms;
   R³, R⁴ and R⁵ each independently are hydrogen or a hydrocarbon group having 1-5 C-atoms; and
   B = a linear or branched oligoglycerol group having from 2 to 20 glycerol units; and
b)(i) at least one cationic or cationizable lipid;
b)(ii) at least one structural lipid; and
b)(iv) at least one PEG-lipid.

In this embodiment, the at least oligoglycerol-containing lipid of Formula (I) may be comprised in the composition in an amount of about 1 mol% to about 25 mol%, preferably about 5 mol% to about 22 mol%, more preferably about 9 mol% to about 21 mol%;
the least one cationic or cationizable lipid may be comprised in an amount of about 40 mol% to about 55 mol%, preferably about 44 mol% to about 52 mol%, more preferably about 46 mol% to about 50 mol%;
the at least one structural lipid in an amount of about 28 mol% to about 52 mol%, preferably about 32 mol% to about 46 mol%, more preferably about 34 mol% to about 44 mol%;
and the least one PEG-lipid in an amount of about 0,5 mol% to about 4 mol%, preferably about 1 mol% to about 3 mol%, more preferably about 1,2 mol% to about 1,7 mol%, based on the total lipid content of the composition.

In a preferred embodiment the present invention relates to a composition comprising:
a) at least one oligoglycerol-containing lipid of Formula (I), 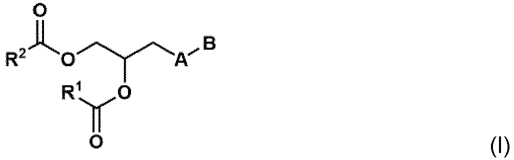
   wherein A = O-P(-O-)(=O)-O; or NR³ ; or N⁺R⁴R⁵ ;
   R¹ and R² each independently are a hydrocarbon group having 12 to 24 C-atoms;
   R³, R⁴ and R⁵ each independently are hydrogen or a hydrocarbon group having 1-5 C-atoms; and
B = a linear or branched oligoglycerol group having from 2 to 20 glycerol units; and
b)(iii) at least one helper lipid.

In this embodiment, the at least oligoglycerol-containing lipid of Formula (I) may be comprised in the composition in an amount of about 20 mol% to about 40 mol%; and the at least one helper lipid may be comprised in the composition in an amount of about 50 mol% to about 80 mol%; based on the total lipid content of the composition.

In some embodiments in the oligoglycerol-containing lipid of Formula (I), B = a linear or branched oligoglycerol group having from 2 to 15 glycerol units, from 2 to 10 glycerol units, from 2 to 5 glycerol units.

In a preferred embodiment the oligoglycerol-containing lipid of Formula (I) is a phosphatidyloligoglycerol, wherein A = O-P(-O-)(=O)-O. Such compounds are also termed DAPGₓ (diacyl-phospho-oligoglycerol; x = number of glycerol units) and in particular DPPGₓ (dipalmitoyl-phospho-oligoglycerol; x = number of glycerol units) or DPPG₂ (dipalmitoyl-phospho-diglycerol) or DPPG₃ (dipalmitoyl-phospho-triglycerol) herein

In a further preferred embodiment, the oligoglycerol-containing lipid of Formula (I) is a cationic oligoglycerol-containing lipid, wherein A = N⁺R⁴R⁵. Such compounds are also termed CLGₓ (cationic lipid containing oligoglycerol; x=number of glycerol units) and in particular CLG₂ (cationic lipid containing diglycerol) herein. Preferably R⁴ and R⁵ each independently are C1-C5-alkyl, and in particular R⁴ = R⁵ = CH₃.

In a further preferred the oligoglycerol-containing lipid of Formula (I) is a cationizable oligoglycerol containing lipid, wherein A = NR³. Such compounds are also termed NLGₓ (cationizable lipid containing oligoglycol; x=number of glycerol units) and in particular NLG₂ (cationizable lipid containing diglycerol) herein. Preferably R³ = C1-C5-alkyl, in particular R³ = CH₃.

According to embodiments of the invention the composition can contain two or more oligoglycerol-containing lipid of Formula (I), preferably at least phosphatidyloligoglycerol, wherein A = O-P(-O-)(=O)-O and at least one cationic oligoglycerol containing lipid, wherein A = N+R⁴R⁵.

Preferably, the at least one phosphatidyloligoglycerol wherin A = O-P(-O-)(=O)-O may be comprised in the composition of the previous embodiments in an amount of about 2 mol% to about 15 mol%, preferably about 4 mol% to about 12 mol%, and the at least one cationic oligoglycerol containing lipid, wherein A = N+R⁴R⁵ is comprised in an amount of about 5 mol% to about 15 mol%, preferably about 8 mol% to about 12 mol% based on the total lipid content of the composition.

Preferred is an oligoglycerol-containing lipid of Formula (I), which comprises from 2 to 4 glycerol units, preferably 2 or 3 glycerol units and more preferably 2 glycerol units.

In the oligoglycerol-containing lipids of Formula (I), residues R¹ and R² each independently are a hydrocarbon group having 12 to 24 C-atoms. Preferred is an oligoglycerol-containing lipid, wherein R¹ and R² independently are a hydrocarbon group having 13 to 19 C-atoms. The hydrocarbon groups can be saturated or mono- or polyunsaturated. The hydrocarbon groups can be linear or branched. Preferably R¹ and R² independently are a linear or branched saturated C13- to C19-alkyl group, more preferably a linear saturated C13- to C19-alkyl group. More preferred, R¹ and R² independently are a linear saturated C15 or C17 alkyl group, most preferred a linear saturated C15 alkyl group.

Most preferred are oligoglycerol-containing lipid is of Formula (II), 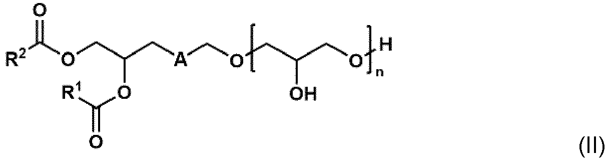 wherein n = 2 to 20; and in particular n = 2 or 3. Such oligoglycerol-containing lipids of Formula (II), have a linear oligoglycerol group and are in particular a di- or triglycerol group.

Most preferred is an oligoglycerol-containing lipid having the Formula 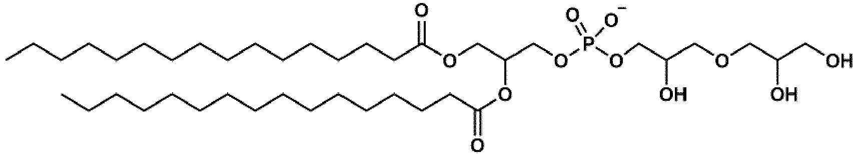 which is termed DPPG₂ herein.

Further most preferred is an oligoglycerol-containing lipid having the Formula 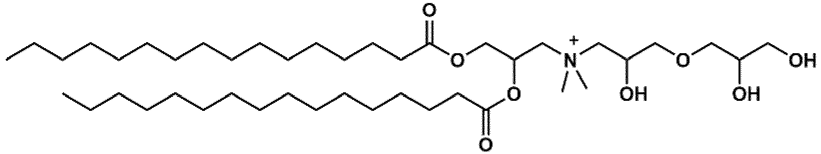 which is termed CLG₂ herein.

Further most preferred is an oligoglycerol-containing lipid having the Formula 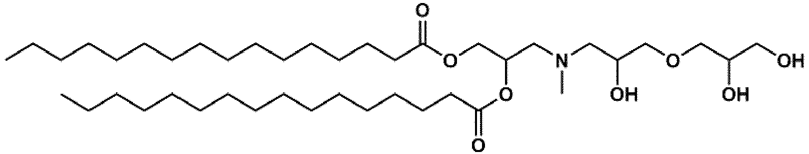 which is termed NLG₂ herein.

A further preferred composition comprises:
(a) at least one oligoglycerol-containing lipid of Formula (I), optionally
   (i) at least one phosphatidyloligoglycerol of Formula (I),
      wherein A = O-P(-O-)(=O)-O; and
   (ii) at least one cationic oligoglycerol-containing lipid of Formula (I),
      wherein A = N⁺R⁴R⁵; and
(b)(i) at least one cationizable lipid; and
(b)(ii) at least one structural lipid.

A further preferred composition comprises:
(a) at least one oligoglycerol-containing lipid of Formula (I); and
(b)(iii) at least one helper lipid, preferably at least two helper lipids.

Particularly preferred the composition is a lipid nanoparticle also referred to as LNP herein.

The term "lipid nanoparticle(s)" or "LNP(s)" as used herein relates to particles characterized by a diameter between 1 and 1000 nm having an outer lipid shell enclosing inner lipid phases. The outer lipid shell may be in the form of a lipid mono- or bilayer. The inner lipid phases may be in the form of one or more lipid monolayers, one or more lipid bilayers, lipid micelles, preferably inverted micelles, and/or solid lipid phases. In a preferred embodiment the lipid nanoparticle comprises at least an outer lipid shell comprised of a lipid monolayer and inner lipid phases comprising inverted micelles.

In a further preferred embodiment, lipid nanoparticle comprises at least an outer lipid shell comprised of a lipid bilayer and inner lipid phases comprising at least one, preferably at least 2, at least 3, at least 4, at least 5 concentrically arranged lipid bilayers. Such LNPs are also referred as multi-layer LNPs herein. Optionally, the concentrically arranged lipid bilayers may further enclose lipid micelles, preferably inverted micelles. The inverted micelles may resemble hexagonal structures.

Furthermore, the spacing between the bilayers may decrease as the curvature increases (see example 9). The multi-layer LNPs may be essentially free of aqueous internal volume.

Further to the lipid constituents, the lipid nanoparticles may comprise water encapsulated by or between the lipid bilayers or the inverted micelles. The LNPs according to the present invention may be characterized by a water content of lower than about 30% (v/v), lower than about 25% (v/v), lower than about 20% (v/v), lower than about 15% (v/v), lower than about 10% (v/v), lower than about 5% (v/v).

The term "lipid nanoparticle" or "LNP" as used herein does not encompass mono-bilayer liposomes comprising only a single lipid bilayer encapsulating an aqueous lumen or liposome in liposome vesicles.

The LNPs according to the invention may have a diameter defined by the Z-average size defined by the intensity weighted mean hydrodynamic size of particles measured by dynamic light scattering (Z-Av) of between about 50 nm to about 1000 nm, between about 70 nm and 700 nm, In one embodiment the diameter may be between about 250 nm and about 550 nm. In another embodiment the diameter may be between about 70 and about 180 nm, or between about 80 nm and 150 nm.

The LNPs according to the invention may have a negative zeta potential, preferably between about -1 and -30 mV.

The compositions according to the present invention comprise diacyl-phospho-oligoglycerols (DAPGₓ), in particular, diacyl-phospho-oligoglycerols having two or three glycerol units, such as dipalmitoyl-phospho-diglycerol (DPPG₂) and dipalmitoylphosphotriglycerol (DPPG₃), as anionic phospholipids as well as their ionizable (NLGₓ, in particular NLG₂) and cationic derivatives (CLGₓ, in particular CLG₂) as a new class of lipids for providing LNPs for encapsulating RNA molecules. The most preferred lipids carry two or three glycerol moieties as head groups, respectively, favourable for design and manufacturing of RNA containing LNPs.

The RNA molecules preferentially comprise protein coding mRNA, non-coding RNA e.g., as immunoadjuvant, siRNA for gene silencing as well as RNA for *in vivo* gene editing, as for example by the CRISPR/Cas technology.

So, diacyl-phospho-oligoglycerols having two or three glycerol units, and in particular DPPG₂ and DPPG₃ as well as their cationic derivatives and their derivatives with the same headgroup but different fatty acid chain lengths, present a new class of lipids (the G₂- and G₃- lipid class) for replacement of PEG lipids or other shielding agents in RNA-LNP formulations. These G₂- and G₃- lipids allow the design of novel next generation RNA-LNP formulations with improved *in vivo* and *in vitro* transfection after parenteral administration, showing low aggregation and high chemical and physical shelf-life stability.

A further advantage of LNPs functionalized with a member of the G₂- or G₃-lipid family is their well-defined and controlled hydrodynamic diameter. For *in vivo* applications the controllable size and charge of the G₂- and G₃-lipid nanoparticles offers the potential to optimize the biodistribution of LNPs.

Further, *in vivo* some target cells are not easily accessible. These target cells might be covered by extracellular matrix molecules, cellular debris, or mucus in the case of pulmonary administration. A small size is also a prerequisite to reach target cells behind the endothelial basement membrane as for example smooth muscle cells or tumor cells by diffusion through pores in the tumor blood vessel system (enhanced permeability and retention [EPR] effect).

A further advantage of the G₂- and G₃-lipids is the potential of their cationic derivatives (e.g., CLG₂) or anionic derivative (e.g., DPPG₂ or DPPG₃) to modulate the overall charge of LNPs, including PEGylated LNPs, as measured by the zeta potential. The charge of LNPs is known to directly modulate their target specificity, e.g., cationic LNPs preferentially accumulate in proliferating endothelial cells. Further the so-called protein corona of the LNPs can be modified by modulating their surface charge, thus indirectly changing their target specificity. So, the G₂- and G₃-lipids represent novel tools for LNP engineering and optimization of their biological activity for treatment of diseases.

A further advantage of the di- and tri-glycerol carrying lipid family is their suitability to produce uniquely compact LNPs (also referred to as multi-layer LNP herein, see also Example 9). EM-pictures showed that the particles are, even without nucleic acid, stable LNP-like structures which are morphological similar to RNA-LNP structures described by (Arteta, et al. 2018), reviewed by (Schoenmaker, et al. 2021), where the outer shell of the LNP consists of one or more lipid layers surrounding an inner core of non-lamellar structure containing the RNA.

In addition to the biophysical findings described above, we have shown that LNPs containing DPPG₂ allow successful entrapment of RNA (e.g., mRNA, siRNA) into uniform and small LNP, and that these LNPs are mechanically and chemically stable, tightly packed vesicles, most probable due to the cooperative effects between the individual lipid bilayers (see examples).

This particular property of G₂ lipid and therefore of nucleic acid-containing LNPs is most likely also based on the property of G₂ or G₃ lipids to displace water from the membranes' interstitial space through interaction with other G₂ or G₃ lipids, especially at stronger curvature. The reduced amount of water between the membranes is expected to result in reduced hydrolysis of the nucleic acid, which is structurally weakened by the electrostatic interaction (the electrostatic interaction between phosphate and a positively charged function destabilizes nucleic acids, since the natural conformation, which is based on repulsion of the phosphate groups, among other things, is thus no longer given). Thus, LNPs with G₂ or G₃ lipids are most likely more stable and could simplify the development of a lyophilized RNA LNP product.

This stabilizing effect is probably further supported by a mechanical effect, the low-water environment enhancing a direct polar interaction of the nucleic acid with the G₂/G₃ groups, so that mechanical influences become less important during LNP production (tight, less flexible wrapping of the nucleic acids).

A further advantage of the G₂- and G₃-lipid containing RNA-LNPs is the avoidance of the potential risk of anaphylactic reactions. There is an ongoing discussion as to whether PEG is causing the few instances of anaphylactic reactions that have been observed with both the Moderna and the Pfizer COVID-19 vaccines. Although the contribution of PEG to the anaphylactic reactions is speculative it is important to investigate whether a next generation of "PEG-free" LNPs will result in different outcomes.

A further advantage of the G₂- and G₃-lipid containing RNA LNPs is the lower potential risk of accelerated blood clearance after repeated administrations. It has been reported that DPPG₂ containing liposomes were not able to induce an immune response against DPPG₂ even after several intravenous administrations (Lokerse, et al. 2021).

The inventive composition preferable comprises at least one cationic or cationizable lipid.

A cationic lipid is typically a lipid having a net positive charge, for example in some embodiments at a certain pH. In some embodiments, a cationic lipid may comprise one or more amine group(s) which bear a positive charge. In some embodiments, a cationic lipid may comprise a cationic, meaning positively charged, headgroup. In some embodiments, a cationic lipid may have a hydrophobic domain (e.g., one or more domains of a neutral lipid or an anionic lipid) provided that the cationic lipid has a net positive charge. In some embodiments, a cationic lipid comprises a polar headgroup, which in some embodiments may comprise one or more amine derivatives such as primary, secondary, and/or tertiary amines, quaternary ammonium, various combinations of amines, amidinium salts, or guanidine and/or imidazole groups as well as pyridinium, piperizine and amino acid headgroups such as lysine, arginine, ornithine and/or tryptophan. In some embodiments, a polar headgroup of a cationic lipid comprises one or more amine derivatives. In some embodiments, a polar headgroup of a cationic lipid comprises a quaternary ammonium. In some embodiments, a headgroup of a cationic lipid may comprise multiple cationic charges. In some embodiments, a headgroup of a cationic lipid comprises one cationic charge.

Preferred cationic lipids are selected from the group comprising DOSPA (2,3-dioleyloxy- N-[2-(sperminecarboxamido)ethyl]- N,N- dimethyl-1-propanaminium trifluoroacetate); DOTAP (1,2-dioleoyl-3-trimethylammonium-propane); DMEPC (1,2-dimyristoyl-sn-glycero-3-ethylphosphocholine); DOTMA (1,2-di-O-octadecenyl-3-trimethylammonium propane); DOTAP (1,2-dioleoyl-3-trimethylammonium propane); DMTAP (1,2-dimyristoyl-3-trimethylammonium propane); DMRIE (2,3-di(tetradecoxy)propyl-(2-hydroxyethyl)-dimethylazanium bromide); DDAB (didodecyl(dimethyl)azanium bromide); DORIE (1,2-dioleyloxypropyl-3-dimethyl-hydroxyethyl ammonium bromide); DC-Choi (3P-[N-(N\N'-dimethylamino-ethane)carbamoyl]cholesterol); DOEPC (dioleyl ether phosphatidylcholine), ePC (ethylphosphatidylcholine).

A cationizable lipid is a lipid that can exist in a positively charged form or neutral form depending on pH. The cationizable lipid may have a pKa value between 5 and 7.5, preferably between 6 and 7. This means that the lipid is uncharged at a pH above the pKa value and positively charged below the pKa value.

Preferred cationizable lipids are selected from the group comprising DLin-MC3-DMA, (6Z,9Z,28Z,31Z)-heptatriaconta-6,9,28,31-tetraen-19-yl 4-(dimethylamino) butanoate); ALC-0315 ((4-hydroxybutyl)azanediyl) bis(hexane-6,1-diyl)bis(2-hexyldecanoate); SM-102 (also termed Lipid H) (heptadecan-9-yl 8-((2-hydroxyethyl) (6-oxo-6-(undecyloxy)hexyl)amino) octanoate); A2-Iso5-2DC18 (ethyl 5,5-di((Z)-heptadec-8-en-1-yl)-1-(3-(pyrrolidin-1-yl)propyl)-2,5-dihydro-1H-imidazole-2-carboxylate); 9A1P9 (decyl (2-(dioctylammonio)ethyl) phosphate); BAME-O16B (bis(2-(dodecyldisulfa-nyl)ethyl) 3,3'-((3-methyl-9-oxo-10-oxa-13,14-dithia-3,6-diazahexacosyl)azanediyl) dipropionate); C12-200 (1,1'-((2-(4-(2-((2-(bis(2-hydroxydodecyl) amino)ethyl) (2-hydroxydodecyl)amino)ethyl) piperazin-1-yl)ethyl)azanediyl) bis(dodecan-2-ol); cKK-E12 (3,6-bis(4-(bis(2-hydroxydodecyl)amino)butyl)piperazine-2,5-dione); OF-Deg-Lin ((((3,6-dioxopiperazine-2, 5-diyl)bis(butane-4,1-diyl))bis(azanetriyl))tetrakis(ethane-2,1-diyl) (9Z,9'Z,9"Z,9"Z, 12Z,12'Z,12"Z,12"Z)- tetrakis (octadeca-9,12-dienoate)); 306Oi10 ((tetrakis(8-methylnonyl) 3,3',3",3"-(((methylazanediyl) bis(propane-3,1diyl))bis (azanetriyl))tetrapropionate)); TT3 ( N1,N3,N5- tris(3-(didodecylamino)propyl)benzene-1,3,5- tricarboxamide); FTT5 ( hexa(octan-3- yl) 9,9',9",9",9"",9""-((((benzene-1,3,5- tricarbonyl)yris(azanediyl)) tris (propane-3,1-diyl)) tris(azanetriyl))hexanonanoate). Preferably the cationizable lipid is selected from ALC-0315 or SM-102.

The inventive composition preferable comprises a structural lipid which is a sterol, preferably selected from the group consisting of cholesterol, fecosterol, sitosterol, ergosterol, campesterol, stigmasterol, brassicasterol, tomadidine, ursolic acid and α-tocopherol; and in particular the structural lipid is cholesterol.

The inventive composition preferable further comprises at least one helper lipid. A helper lipid is a lipid capable of increasing the effectiveness of delivery of lipid-based particles to a target, preferably into a cell. The helper lipid can be neutral or negatively charged. Particular suitable helper lipids are selected from
hydrogenated soy L-α-phosphatidylcholine (HSPC);
1,2-distearoyl-*sn*-glycero-3-phosphocholine (DSPC);
1,2-diolioyl-*sn*-glycero-3-phosphoethanolamin (DOPE);
1,2-dipalmitoyl-*sn*-glycero-3-phosphocholine (DPPC);
1,2-dimyristoyl-*sn*-glycero-phosphocholine (DMPC);
1,2-diolioyl-*sn*-glycero-3-phosphocholine (DOPC); and
1-palmitoyl-2-olioyl-*sn*-glycero-3-phosphocholine (POPC).
1,2-distearoyl-*sn*-glycero-3-phosphocholine (DSPC) is most preferred.

In some embodiments of the invention the composition does not comprise a helper lipid. It was found that helper lipids, such as DSPC, contained in LNPs can be replaced by oligoglycerol-containing lipids of Formula (I) resulting in stable LNPs.

The composition of the invention may further comprise
e) a PEG lipid.

The term "PEG lipid" or "PEGylated lipid" refers to a lipid which is covalently bound to polyethylene glycol (PEG). Polyethylene glycol (PEG) is one of the most widely applied nonionic, hydrophilic polymer that provides the stealth effect for the LNPs improving their circulation time in the body (Knop, et al. 2010) (Albertsen, et al. 2022).

In an embodiment of the invention comprising a PEG lipid only part of PEG lipid included in conventional LNP formulations is replaced by at least one oligoglycerol-containing lipid of Formula (I), and in particular by at least one oligoglycerol-containing lipid of Formula (I), wherein A = O-P(-O-)(=O)-O.

In a further embodiment of the invention comprising a PEG lipid, at least one oligoglycerol-containing lipid of Formula (I), and in particular at least one oligoglycerol-containing lipid of Formula (I), wherein A = NR³ ; or N+R⁴R⁵ is present in addition to the PEG lipid. In those embodiments the at least one oligoglycerol-containing lipid of Formula (I), and in particular at least one oligoglycerol-containing lipid of Formula (I), wherein A = NR³; or N+R⁴R⁵ is used to modulate the LNP composition.

In a most preferred embodiment the composition of the invention does not comprise a PEG lipid.

According to the invention empty carrier systems and in particular empty lipid nanoparticles (LNPs) can be provided. Preferred however are compositions, further comprising
c) a therapeutic and/or prophylactic agent.

Most preferred, the therapeutic and/or prophylactic agent is a nucleic acid, in particular a ribonucleic acid (RNA). The RNA is preferably selected from the group consisting of messenger RNA (mRNA), antisense RNA (aRNA), small interfering RNA (siRNA), CRISPR-RNA (crRNA, tracrRNA or sgRNA), asymmetrical interfering RNA (aiRNA), micro RNA (miRNA), Dicer-substrate RNA (dsRNA), small hairpin RNA (shRNA), and mixtures thereof.

In a further preferred embodiment the therapeutic and/or prophylactic agent is a small molecule, in particular a cytostatic. Preferably the inventive composition comprises doxorubicin (DOX) which is a cytotoxic anthracycline. Further suitable cytostatic anthracyclines presently employed in cancer treatment include epirubicin, idarubicin, daunorubicin and mitoxantrone. In further embodiments of the invention other cytostatics such as mitomycin C, gemcitabine, trabectedin, etc. or platinum derivatives such as cisplatin, carboplatin or oxaliplatin or other agents disclosed below can be included.

In a further preferred embodiment the composition comprises an alternative of additional active pharmaceutical ingredient which is selected from the group consisting of further anthracyclines such as aclarubicin, amrubicin, pirarubicin, valrubicin and zorubicin; anthracenediones such as mitoxantrone and pixantrone; antineoplastic antibiotics such as mitomycin and bleomycin; vinca alkaloids such as vinblastine, vincristine and vinorelbine; alkylating agents such as cyclophosphamide and mechlorethamine hydrochloride: campthothecins such as topotecan, irinotecan (CPT-11), lurtotecan, 9-aminocamptothecin, 9-nitrocamptothecin and 10-hydroxycamptothecin; purine and pyrimidine derivatives such as 5-fluorouracil, gemcitabine (2,2'-difluoro-2-deoxycytidine, dFdC), floxuridine (FUDR), cytarabine (cytosine arabinoside), 6-azauracil (6-AU); oxazaphosphorines such as cyclophosphamide, ifosfamide and trofosfamide; taxanes such as paclitaxel and docetaxel; podophyllotoxin derivatives such as etopside and teniposide; platinum-based compounds such as cisplatin, carboplatin, oxaliplatin, nedaplatin; methotrexate; tyrosine kinase inhibitors such as imatinib, gefitinib, erlotinib, sunitinib, adavosertib and lapatinib; and cytarabines such as cytosine arabinoside.

Most preferred according to the invention is a composition comprising:
a) at least one cationic or cationizable lipid;
b) cholesterol;
c) at least one of 1,2-dipalmitoyl-sn-glycero-3-phosphodiglycerol and 1,2-dipalmitoyl-*sn*-glycero-3-phosphotriglycerol;
d) 1,2-distearoyl-*sn*-glycero-3-phosphocholine (DSPC); and
e) optionally a therapeutic and/or prophylactic agent.

This composition preferably does not contain a PEG lipid.

Further most preferred according to the invention is a composition comprising:
a) at least one cationic or cationizable lipid;
b) cholesterol;
c) at least one of 1,2-dipalmitoyl-*sn*-glycero-3-phosphodiglycerol and 1,2-dipalmitoyl-*sn*-glycero-3-phosphotriglycerol;
d) CLG₂; and
e) optionally a therapeutic and/or prophylactic agent.

This composition preferably does not contain a PEG lipid.

Further most preferred according to the invention is a composition comprising:
a) at least one of 1,2-dipalmitoyl-*sn*-glycero-3-phosphodiglycerol and 1,2-dipalmitoyl-*sn*-glycero-3-phosphotriglycerol;
b) at least one of 1,2-distearoyl-*sn*-glycero-3-phosphocholine (DSPC) and 1,2-dipalmitoyl-*sn*-glycero-3-phosphocholine (DPPC); and
c) optionally a therapeutic and/or prophylactic agent.

This composition preferably does not contain a PEG lipid.

Further most preferred according to the invention is a composition comprising:
a) at least one cationic or cationizable lipid;
b) cholesterol.
c) CLG₂; and
d) a PEG lipid, and
e) optionally a therapeutic and/or prophylactic agent.

In preferred embodiment the invention relates to multi-layer LNPs comprising at least an outer lipid shell comprised of a lipid bilayer and inner lipid phases comprising at least one, preferably at least 2, at least 3, at least 4, at least 5 concentrically arranged lipid bilayers. Preferably the concentrically arranged lipid bilayers further enclose lipid micelles, preferably inverted micelles (hexagonal structures) and comprising any of the compositions described herein.

In a preferred embodiment the multi-layer LNPs as described above comprises:
a) at least one oligoglycerol-containing lipid of Formula (I), 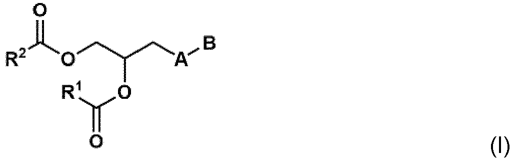
   wherein A = O-P(-O-)(=O)-O; or NR³ ; or N⁺R⁴R⁵ ;
   R¹ and R² each independently are a hydrocarbon group having 12 to 24 C-atoms;
   R³, R⁴ and R⁵ each independently are hydrogen or a hydrocarbon group having 1-5 C-atoms; and
   B = a linear or branched oligoglycerol group having from 2 to 20, preferably 2 to 5, glycerol units; and
b)(iii) at least one helper lipid, most preferably two helper lipids,

Preferably, the oligoglycerol-containing lipid of Formula (I) is selected from DPPG₂ or DPPG₃, most preferably DPPG₂, and comprises DPPC and DSPC as helper lipids.

DPPG₂ and/or DPPG₃ is preferably comprised in an amount of at least 2 mol%, more preferably at least 5 mol%, based on the lipid content of the multi-layer LNP. Such LNP are in particular suitable for nucleic acid entrapment, but can also include other drugs such as cytostatic, lipophilic and also hydrophilic agents (e.g., doxorubicin, gemcitabine) in particular as given herein. A further aspect is such a LNP for treating a disease, in particular for treatment of cancer, for cancer immunotherapy, for treatment of infectious diseases and/or for prevention of infectious diseases.

In a preferred embodiment, multi-layer LNPs may preferably comprise a lipid mixture of DPPC/DSPC/DPPG₂ in a molar range of about 40 to about 60 mol% DPPC, about 15 to about 25 mol% DSPC and about 20 to about 40 mol% DPPG₂, based on the total lipid content of the composition.

The inventive compositions and in particular inventive DPPG₂ or DPPG₃ based LNPs are particularly suitable for drug delivery.

In a further preferred embodiment, the present invention relates to a composition comprising:
a) at least one oligoglycerol-containing lipid of Formula (I), 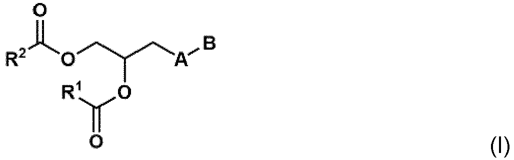
   wherein A = O-P(-O-)(=O)-O; or NR³ ; or N⁺R⁴R⁵ ;
   R¹ and R² each independently are a hydrocarbon group having 12 to 24 C-atoms;
   R³, R⁴ and R⁵ each independently are hydrogen or a hydrocarbon group having 1-5 C-atoms; and
   B = a linear or branched oligoglycerol group having from 2 to 20 glycerol units; and
b)(iii) at least one helper lipid;
b)(ii) at least one structural lipid; and
b)(iv) at least one PEG-lipid;
wherein the oligoglycerol-containing lipid of Formula (I) is NLG₂ or CLG₂.

Preferably the structural lipid is cholesterol, the helper lipid is DSPC and the PEG-lipid is DSPE-PEG. Preferably NLG₂/cholesterol/DSPC/DSPE-PEG in a molar range of about 35 mol% to about 55 mol% NLG₂, about 30 mol% to about 50 mol% cholesterol, about 5 mol% to about 15 mol% DSPC and about 0 mol% to about 6.5 mol% DSPE-PEG, more preferably about 42 mol% to about 50 mol% NLG₂, about 39 mol% to about 47 mol% cholesterol, about 8 mol% to about 12 mol% DSPC and about 1 mol% to about 2 mol% DSPE-PEG, based on the total lipid content of the composition.

Preparation of LNPs so far bases on a microfluidic approach in which an aqueous phase, typically with a low pH and containing a nucleic acid, is rapidly mixed with an ethanolic solution the necessary lipids, typically including PEG-lipids, cholesterol, DSPC and an ionizable lipid, which has a positively charge at low pH-values. The resulting primary dispersion of smaller liposome-like vesicles containing the nucleic acids are then dialyzed against a buffer having neutral pH which neutralizes the positive charge of the vesicles. Therefore, electrostatic repulsion no longer keeps the vesicles at a distance and causes vesicle fusion. This fusion stop when a particle size is reached with the maximum density of PEG-chains on its surface. Thus, the ratio of PEG-lipid in the lipid mixture determined the final LNP sizes.

The LNPs may be produced without prior art methods including for example microfluidic methods or organic solvent injection or using dual centrifugation (DC) without organic solvents (e.g., ethanol).

Most preferably the multi-layer LNPs as described above may be produced comprising a process described herein which comprises a dual centrifugation (DC). Preparation of lipid comprising particles using DC is for example described by Massing et. al 2017 or Koehler et al. 2023. Dual centrifugation includes rotation of samples around a central axis, as in conventional centrifugation, and additionally rotating the samples around a second rotation axis. Due to that the direction of high centrifugal acceleration in the sample vials changes continuously, which result in high frequent and very powerful sample movements.

In a general aspect, the invention relates to a process for providing multi-layer LNPs wherein an aqueous composition comprising a total lipid concentration of at least about 50 % (w/v), preferably at least about 55 % (w/v), more preferably at least about 60 % (w/v) is subjected to dual centrifugation.

The process for preparing multi-layer LNPs comprising a DC may comprise at least the following steps in the disclosed sequence:

| | |
|---|---|
| Step 1 | comprising providing a first composition comprising a lipid mixture in a first aqueous medium, and preferably beads; |
| Step 2 | comprising subjecting the first composition to dual centrifugation to obtain an intermediate composition; |
| Step 3 | comprising diluting the intermediate composition obtained in Step 2 with a dilution medium to obtain a diluted composition; |
| Step 4 | comprising subjecting the diluted composition obtained in Step 3 to a second dual centrifugation to obtain a composition comprising multi-layer LNPs. |

The intermediate composition may be a vesicular phospholipid gel (VPG).

Generally, the first aqueous medium and the dilution medium may be selected according to the lipids in the lipid mixture. For example, the first aqueous medium and/or the dilution medium may be a buffered aqueous medium having a pH between about 7.0 and about 9.0. The first aqueous medium may comprise further agents such as at least one chelator, for example EDTA, at least one salt, for example NaCl and/or at least one reducing agent, for example sodium dithionite.

In Step 3 the intermediate composition may be diluted with dilution medium in a (v/v) ratio of intermediate composition:dilution medium of about 1 to about 3.5:1, preferably 1.5 to about 2.5:1.

In an embodiment wherein the lipid mixture comprises a cationizable lipid, such as for example NLG₂, the first aqueous medium is preferably an acidic buffered aqueous medium having a pH between about 3.0 and about 5.0, preferably about 4.0 and the dilution medium is a buffered aqueous medium having a pH between about 7.0 and about 8.0, preferably about 7.4, Preferably the dilution medium has a higher buffer capacity than the first aqueous. Accordingly, the dilution in Step 3 leads to a neutralization of the diluted composition.

In a preferred embodiment, the first composition employed in Step 1 comprises lipids according to mixtures of lipids as described above. Preferably, first said composition comprises a total lipid concentration of at least about 50 %(w/v), at least about 55 %(w/v), at least about 60 %(w/v) at least about 65 %(w/v). The unit "%(w/v)" as used herein with regard the first composition defines the mass of lipids in relation to the aqueous medium. Accordingly, a lipid concentration of 50 %(w/v) refers to a composition comprising 50 mg lipids and 50 µl aqueous medium and a lipid concentration of 60 %(w/v) refers to a composition comprising 60 mg lipids and 40 µl aqueous medium.

In a further aspect, the invention relates to multi-layer LNPs obtained or obtainable by the process disclosed herein.

In a preferred embodiment, a drug substance to be encapsulated into the multi-lamellar LNPs, such as a small molecule or nucleic acid, for example RNA, is comprised in first composition employed in Step 1, or added during Step 3, preferably comprised in the dilution buffer.

In a preferred embodiment, first composition employed in Step 1 may comprise a polyalcohol. The polyalcohol may preferably be selected from a disaccharide, for example sucrose and/or trehalose, a sugar alcohol, for example mannitol, or glycerol.

Formulation of LNPs by DC provides a time efficient and convenient method of preparation. Advantageously, the use of organic solvents and the lengthy purification methods like dialysis (for elimination of organic solvent) can be excluded. The invention also allows for the preparation of patient-specific LNPs (e.g., with patient-specific mRNA to support cancer immune therapy) using DC. Bedside DC-preparation allows the application of LNPs that may not be perfectly storage stable, furthermore kits containing the necessary vials, lipids and drugs can be made available.

The morphology of the multi-layer LNPs are shown in Example 9. The multi-layer LNPs according to the invention may have a polydispersity index (PDI) between about higher than 0.01 of lower than about 0.25, lower than about 0.2, lower than about 0.15, lower than about 0.1. The polydispersity index (PDI) may for example be higher than 0.005.

The low PDI of the multi-layer LNPs may further be maintained over time since the multi-layer LNPs do not tend to aggregate. Virtually no LNP aggregation due to LNP-LNP-interactions takes place due to PG₂-headgroup-interactions between PG₂-lipids on the surface of two neighbor LNPs, which may be explained by the bulk water outside the vesicles. The water molecules form hydrogen-bonds to the hydroxyl-groups of the G₂-headgroups and thus prevent interactions of neighboring G₂-headgroups. The addition of poly-alcohols like sugars, glycerol, PEG of different lengths and some more are able to further diminish PG₂/G₃-LNP or - Liposome aggregation

The Z-average size of the multilayer-LNPs as characterized by the intensity weighted mean hydrodynamic size of particles measured by dynamic light scattering (Z-Av) is preferably lower than about 200 nm, lower than about 150 nm, lower than about 140 nm, and or preferably at least about 80 nm, at least about 90 nm, at least about 100 nm, at least about 110 nm. The average size may be between about 200 nm and about 80 nm, between about 150 nm and about 100 nm. Accordingly, the multi-layer LNPs according to the invention are in the preferred size range with a very narrow size distribution for various *in vivo* applications routes.

Surprisingly the multi-layer LNPs derived according to the invention are unusually tightly packed in the above described structure comprising an outer lipid shell comprised of a lipid bilayer and inner lipid phases comprising concentrically arranged lipid bilayers which enclose inverted micelles (hexagonal structures). The multi-layer LNPs may be essentially free of an aqueous internal volume. In this context of the present disclosure "essentially free" refers to about less than 10%(v/v), about less than 5%(v/v), about less than 2%(v/v).

This was insofar surprising as lipid mixtures other than described above, for example hydrogenated egg PC/cholesterol with and without PEG lipids, clearly result in particles with a different morphology (Koehler, et al. 2023). Since this is also the case for pure DPPC, the formation of the newly discovered multi-layer LNPs bases is the result of very special properties of DPPG₂/₃ as part of the lipid mixture.

It was also surprisingly found that, despite obviously enforced by the chosen preparation conditions such as high lipid concentration and a shortage of water, the new multi-layer LNPs are surprisingly stable regarding their morphology and chemical stability of the lipid excipients, even after their dilution in an excess of water.

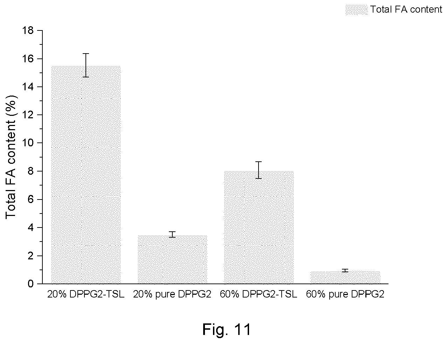

As shown in Figure 11 and Example 9, the tightly packed multi-layer LNPs obtained by the process described above by DC from high concentration lipid solutions exhibit a significantly reduced lipid, including DPPG₂/₃, hydrolysis (fatty acid release) in comparison to small unilamellar vesicles (SUVs) comprising the same lipid mixture. The anti-hydrolytic effect in the multi-layer LNPs may be increased by the addition of polyalcohols, such as sucrose, trehalose, mannitol, or glycerol in solution 1 of the method for preparing multi-layer LNPs according to the invention.

This stability seems to be a very special characteristic of the lipid DPPG₂/₃ as part of the lipid mixture. Without wishing to being bound by theory, the inventors assume that this very special behavior of DPPG₂ or DPPG₃ lipids is most probably due to an interaction between the oligoglycerol-headgroups due to polar and protic interaction (hydrogen bonding) because of their free hydroxyl groups.

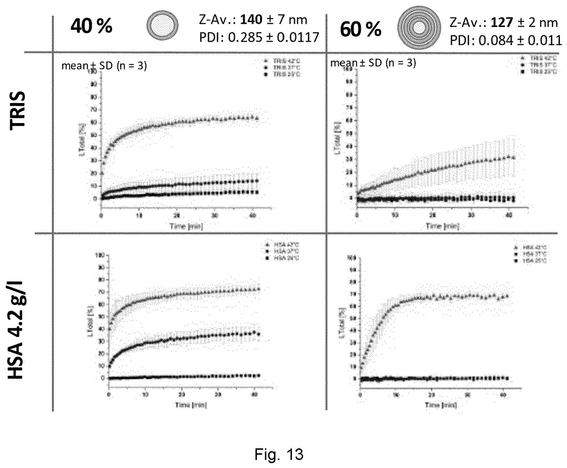

That such an advantageous behavior has not been observed for PEG-carrying lipids can be explained by the fact that the ethylene glycol moiety of usually used PEG-lipids can only interact with water, but not with both, water as well as other PEG -lipids. In contrast to PEG, DPPG₂ or DPPG₃ lipids have free hydroxyl-groups which can interact via hydrogen-bonding with the hydroxyl-groups of neighboring DPPG₂ or DPPG₃ lipids or with DPPG₂ or DPPG₃ lipids of the opposing, neighbor membranes. Since membranes containing DPPG₂ or DPPG₃ lipids with packing parameters of > 1 tend to form rather planar membranes, it was surprising that the newly discovered cooperative effects between the DPPG₂ or DPPG₃ containing membranes forces the membranes to form perfectly round particles, an effect. The new morphology which stabilizes those multi-layer LNPs also renders them more stable against membrane interactions with serum proteins and successfully eliminated the unwanted leakage of encapsulated compounds at body temperature as known for classical thermosensitive liposomes (shown by the comparison of calcein release from DPPG₂-containing multi-layer LNP with unilamellar vesicles with and without presence of albumin, respectively) (Figure 13).

That PEG-containing liposomes are, in contrast to DPPG₂/G₃-containing particles not prone to form LNPs can be illustrated by comparing EMI images of liposomes (small multilamellar vesicles, SMV) containing normal phospholipids/cholesterol with and without PEG and, in comparison, liposomes containing DPPG₂. The smallest membrane spacing is found in DPPG₂ liposomes, and spacing decreases further with increasing curvature. Normal and equal spacing across all membranes is found in phosphatidylcholine/cholesterol liposomes with and without PEG-lipids. PEG-containing liposomes have also much fewer lamellae, which have significantly larger spacing).

Without wishing to being bound by theory, the inventors further assume that the stability of the LNPs described herein may further be supported by a mechanical effect. The low-water environment may enhance a direct polar interaction of the to be encapsulated compound (e.g., nucleic acid) with the oligoglycerol groups, so that mechanical influences may become less important during LNP production (e.g., tight, less flexible wrapping of the nucleic acids). Thus, multi-layer LNPs with oligoglycerol lipids may be more stable and simplify the development of lipid-based pharmaceutical formulations (e.g., lyophilized RNA-LNP).

The described multi-layer LNPs may have a high entrapment efficiency (EE) for watersoluble substances which, in addition to the entrapment of nucleic acids (e.g., RNA, siRNA, mRNA, DNA), also efficiently and economically enables the entrapment of other watersoluble active pharmaceutical ingredients.

The invention also allows for the preparation of patient-specific LNPs (e.g., with patient-specific mRNA to support cancer immune therapy) using DC. Bedside DC-preparation allows the application of LNPs that may not be perfectly storage stable, furthermore kits containing the necessary vials, lipids and drugs can be made available.

### Figures

The present invention is further illustrated by the enclosed figures.
Figure 1: Potency of NLG₂ containing LNPs is compared to transfection (luminescence units) of the PEG containing Formulation B (positive control). Luminescence was measured 24 h after transfection of HEK 293 cells in serum containing medium. Luciferase mRNA was encapsulated into LNPs containing varying ratios of NLG₂.
Figure 2: Agarose gel electrophoresis for detection of the mRNA encapsulation efficiency of different LNPs. PEG-containing LNP Formulation B was compared to the RNA-LNPs with DPPG₂ as the shielding lipid. (+ T = Triton treated)

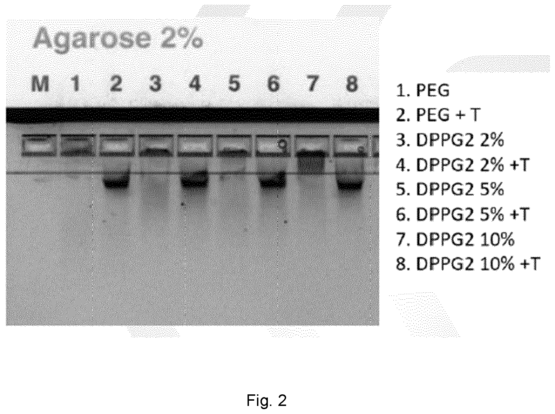

Figure 2: Potency of DPPG₂ containing LNPs (based on Formulation B w/o PEG) is presented as x-fold transfection (luminescence units) of the LNP Formulation B which acts as positive control. GFP mRNA was encapsulated into LNPs with varying DPPG₂ contents. Luminescence was measured 24 h after transfection of HEK 293 cells in serum containing medium. Error bars are SD.

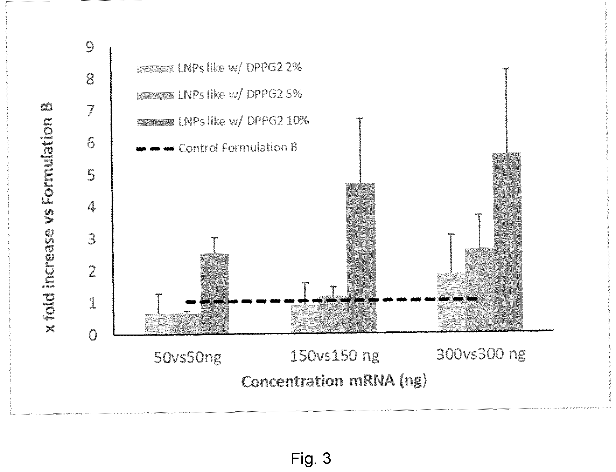

Figure 3: Potency of DPPG₂ containing LNPs (based on Formulation B w/o PEG) without DMG-PEG is presented as absolute luminescence units compared to Formulation B. Luminescence measured 24 h after transfection of HEK 293 cells with LNPs containing GFP mRNA. Error bars are SD.

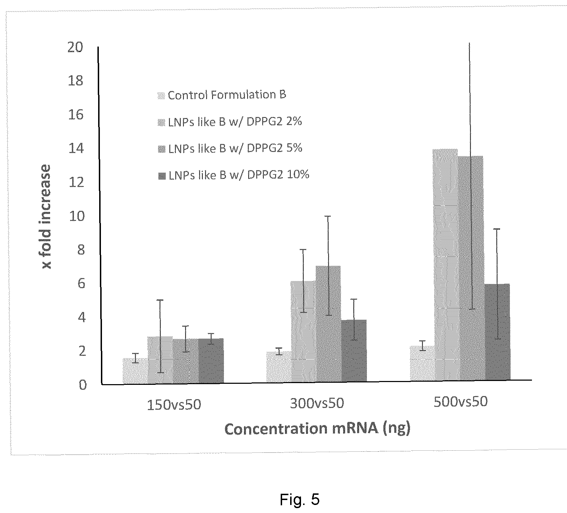

Figure 5: Luminescence of the different DPPG₂ LNPs containing 150, 300 and 500 ng mRNA is presented as x-fold transfection (luminescence units) of the respective DPPG₂ formulations containing 50 ng mRNA. Luminescence was measured 24 h after transfection of HEK 293 cells with GFP mRNA encapsulated into LNPs with varying DPPG₂ contents. DPPG₂ formulations were based on Formulation B without PEG. Formulation B served as positive control. Error bars are SD.

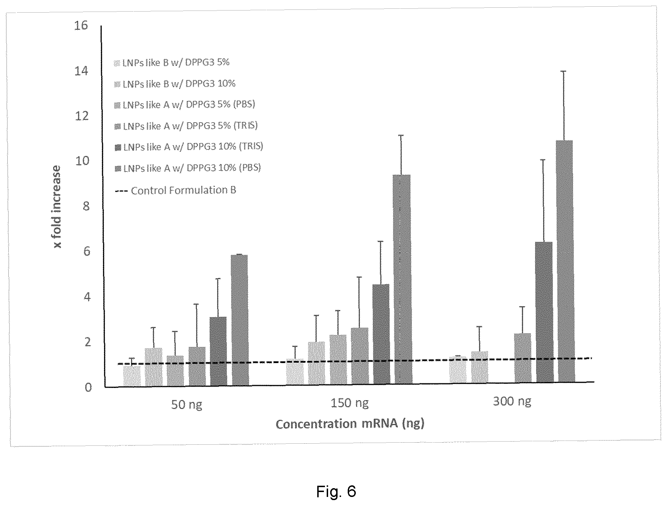

Figure 6: Potency of DPPG₃ containing LNPs is presented as x-fold transfection (luminescence units) of the LNP Formulation B which acts as positive control. Luminescence was measured 24 h after transfection of HEK 293 cells with serum containing medium. Luciferase mRNA was encapsulated into LNPs with varying DPPG₃ contents. Error bars are SD.

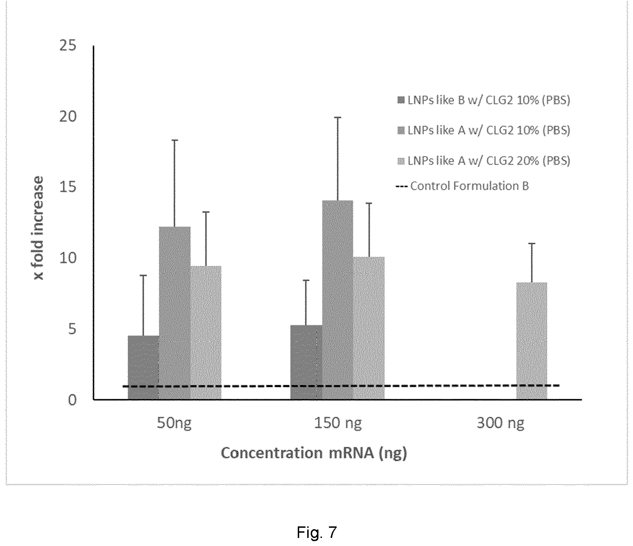

Figure 7: Potency of CLG₂ containing LNPs is presented as x-fold transfection (luminescence units) of the Formulation B (positive control). Luminescence was measured 24 h after transfection of HEK 293 cells with serum containing medium. Luciferase mRNA was encapsulated into LNPs with varying CLG₂ contents. LNPs compositions were based on Formulation A and Formulation B, respectively (see text for details). Error bars are SD.

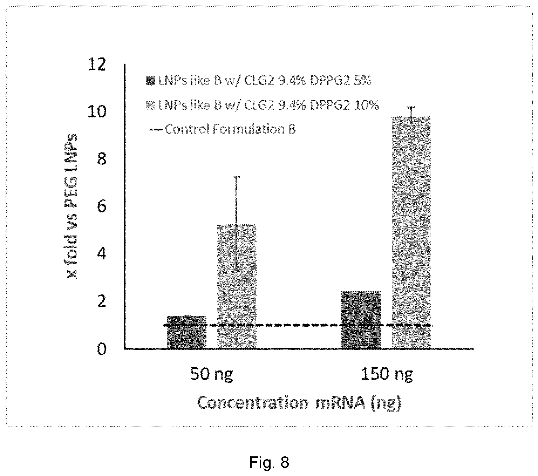

Figure 8: Potency of CLG₂ and DPPG₂ containing LNPs is presented as x-fold transfection (luminescence units) of Formulation B (positive control). Formulation B contains DSPC as helper lipid and DMG-PEG as shielding lipid. Luminescence was measured 24 h after transfection of HEK 293 cells with serum containing medium. Luciferase mRNA was encapsulated into LNPs with varying DPPG₂ contents. Error bars are SD.

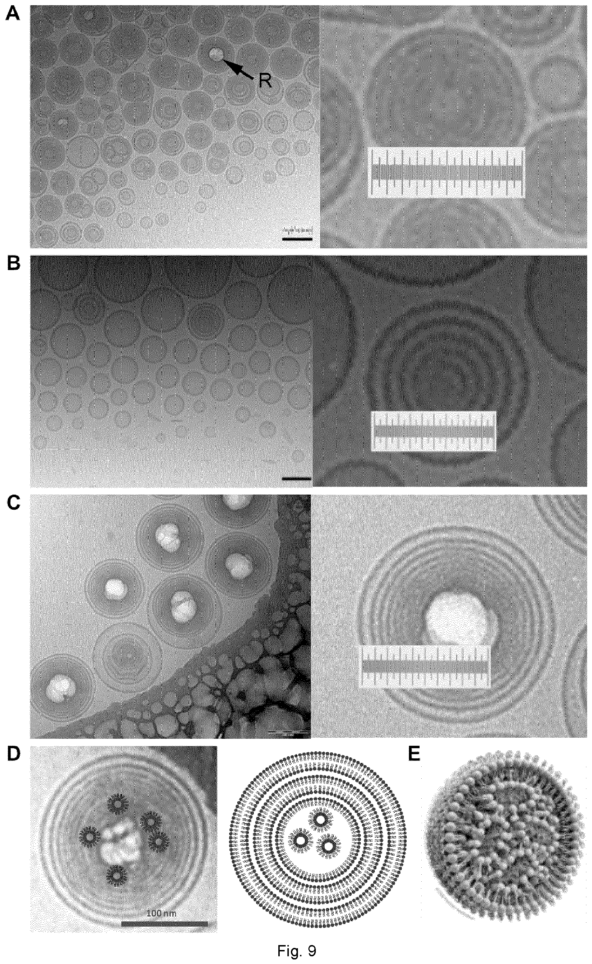

Figure 9: Morphology of various lipid mixtures after DC-homogenization. (A) HEPC/cholesterol 55/45 (mol/mol) small multilamellar vesicles (SMVs) show multiple lamellae surrounding a small aqueous core. The distance of the lamellae is 3-4 nm and always constant (scale bar 100 nm). (B) HEPC/cholesterol/DSPE-PEG2000 50/45/5 (mol/mol) SMVs show multiple lamellae surrounding a small aqueous core. The number of lamellae is lower compared to the PEG-free HEPC/cholesterol SMVs. The distance between the lamellae is 5-6 nm and always constant (scale bar 100 nm). (C) Novel multi-layer LNPs made of DPPG₂/DSPC/DPPC 50:20:30 (mol/mol) show multiple lamellae surrounding a core completely filled with lipids, probably a hexagonal phase or inverted micelles. The number of lamellae is higher compared to the PEG-free HEPC/cholesterol SMVs, the distance between the lamellae is surprisingly not constant but gets smaller the higher the curvature is. The distance between the outer and the second layer is the lamellae is 5-6 nm, the next distance is 3-4 nm (scale bar 100 nm). (D) Schematic representation of multi-layer LNPs. (E) Cartoon of a proposed structure of LNPs (source: www.precisionnanosystems.com). (R) Indicate radiation damage.

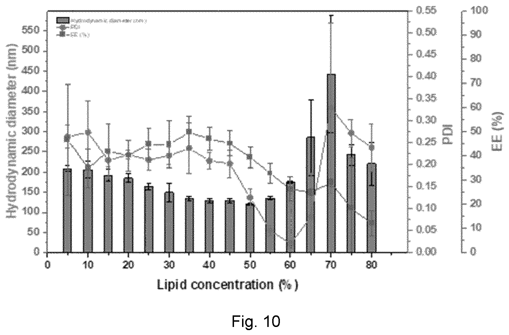

Figure 10: Liposomes and LNPs made of DPPG₂, DSPC and DPPC using dual centrifugation (DC) at various lipid concentrations. LNPs can be found at concentrations around 60 %, where PDI values go significantly down, below 50% more liposomes than LNPs were formed. It could be shown that even at 60 % lipid concentration, the entrapping efficiency for water soluble molecules (here: calcein) is high and still around 30 %.
Figure 11: Chemical stability of DPPG₂-TSL during DC-preparation for DPPGz-hydrolysis (fatty acid formation) at different lipid concentrations was compared to the stability of vesicles only formed by DPPG₂ at the same lipid concentration (%). Data are shown as the mean ± SD (n=3).

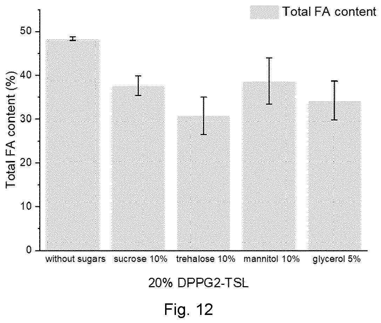

Figure 12: Anti-hydrolytic effects of poly-alcohols on DPPG₂-TSL during DC-homogenization (60 min). Fatty acid formation was measured by HPTLC. Data are shown as the mean ± SD (n=3).
Figure 13: Release of entrapped model drug and fluorescent dye calcein from DPPG₂-based liposomes (left) and DPPG₂-based multi-layer LNPs after incubation at room temperature (RT), 37°C as well as 42°C, respectively. Incubation was performed either with or without albumin.

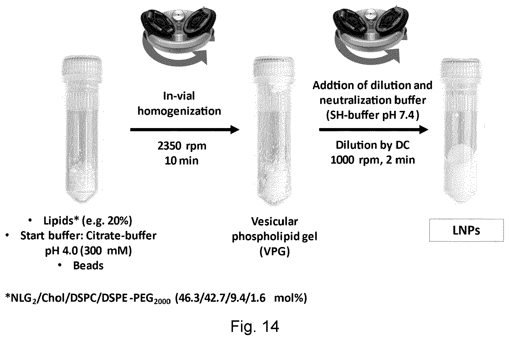

Figure 14: Graphical presentation of preparation of LNPs from NLG₂ lipids using dual centrifugation.

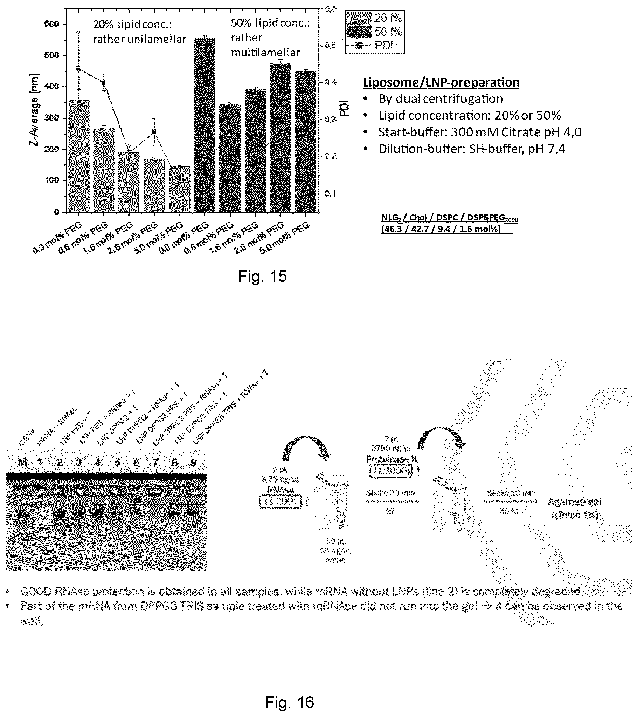

Figure 15: Preparation of LNPs by DC using 20% vs 50% lipid concentration as well as various ratios of PEG-PE. With 20% lipid concentration, predominantly SUV have been formed by DC, which form after neutralization larger particles. SUV particle sizes strictly dependent on the amount of PEG-PE in the lipid blend, which is in accordance with previous findings of other groups. With 50% lipid concentration, predominantly multilamellar structures (LNP) have been formed by DC. Those LNPs are bigger and due to cooperative effects between the membranes more stable by themselves and thus do not aggregate, independent of the ratio of PEG-lipid in the lipid mixture.
Figure 16: Agarose gel assay used to demonstrate that the DPPG₂ containing RNA-LNPs based on the formulation B could protect encapsulated mRNA loads when exposed to RNase.

### List of references

Albertsen, Camilla Hald, Jayesh Kulkarni, Dominik Witzigmann, Marianne Lind, 'Karsten Pertersson, und JensB. Simonsen. "The role of lipid components in lipid nanoparticles for vaccines and gene therapy." Advanced drug delivery reviews 188 (2022): 114416-114428.
Ambegia, E., S. Ansell, P. Cullis, J. Heyes, L. Palmer, und I. MacLachlan. "Stabilized plasmid-lipid particles containing PEG-diacylglycerols exhibit extended circulation lifetimes and tumor selective gene expression." Biochimica et Biophysica Acta 1669 (2005): 155-163.
Arteta, Yanez, et al. "Successful reprogramming of cellular protein production through mRNA delivered by functionalized lipid nanoparticles." PNAS 115 (2018): E3351-E3360.
Brockow, Knut, et al. "Experience with polyethylene glycol allergy-guided." Allergy 77 (2022): 2200-2210.
Edo Kon, Uri Elia, Dan Peer,. "Principles for designing an optimal mRNA lipid nanoparticle vaccine,." Current Opinion in Biotechnology, 2022.
Garcia, Josep, Carles Felip, Svea Stephan, and Paul Pietsch. Development and optimization of LNP formulations- using Knauer Nanoscaler. White paper - Curapath, 2022.
Hattori, Yoshiyuki, Kyoko Tamaki, Sho Sakasai, Kei-Ichi Ozaki, und Hiraku Onishi. "Effects of PEG anchors in PEGylated siRNA lipoplexes on in vitro genbe silencinf effects and siRNA biodistribution in mice." Molecular Medicine Reports 22 (2020): 4183-4196.
Heyes, James, Kim Hall, Vicky Tailor, Richard Lenz, und Ian MacLachlan. "Synthesis and characterization of novel polyethylene glycol)-lipid conjugates suitable for use in drug delivery." Journal of controlled release 112 (2006): 280-290.
Hou, Xucheng, Tal Zaks, Robert Langer, und Yizhou Dong. "Lipid nanoparticles for mRNA delivery." Nature Reviews Materials 6 (2021): 1078-1094.
Jaggers, Jordon, and Anna R. Wolfson. "mRNA COVID-19 Vaccine Anaphylaxis: Epidemiology, Risk Factors,." Current Allergy and Asthma Reports 23 (2023): 195-200.
Knop, Katrin, Richard Hoogenboom, Dagmar Fischer Prof. Dr., und Ulrich S. Schubert Prof. "Poly(ethylene glycol) in Drug Delivery: Pros and Cons as Well as Potential Alternatives." Angewandte Chemie International Edition 49 (2010): 6288-6308.
Koehler, Jonas K., et al. "Tailoring the Lamellarity of Liposomes Prepared by Dual Centrifugation." pharmaceutics 15 (2023): 706-727.
Kulkarni, Jayesh, Dominik Witzigmann, und Sam Chen. "Lipid Nanoparticle Technology for Clinical Translation of siRNA Therapeutics." Accounts of Chemical Research, 2019.
Li, Q., et al. "RNA editing underlies genetic risk of common inflammatory diseases." Nature 608 (2022): 569-577.
Lokerse, Wouter J.M., et al. "Mechanistic investigation of thermosensitive liposome immunogenicity and understanding the drivers for circulation half-life: A polyethylene glycol versus 1,2-dipalmitoyl-sn-glycero-3-phosphodiglycerol study." Journal of controlled release 333 (2021): 1-15.
Nogueira, Sara S., et al. "Polysarcosine-Functionalized Lipid Nanoparticles for Therapeutic mRNA delivery." ACS Applied Nano Materials 3 (2020): 10634-10645.
Petrini, Matteo, Wouter JM Lokerse, Agnieszka Mach, Martin Hossann, Olivia M Merkel, und Lars Lindner. "Effect of surface charge, pegylation and functionalization with Dipalmitoylphosphatidyldiglycerol on Liposome- Cells interactions and Local drug delivery to solid tumors via thermosensitive liposomes." International journal of nanomedicines 16 (2021): 14045-14061.
Picard, Matthieu, et al. "Safety of COVID-19 vaccination in patients with polyethylene glycol allergy: A case series." The Journal of Allergy and Clinical Immunology: In Practice 10 (2022): P620-P625.
Sabnis, Staci, et al. "A Novel Amino Lipid Series for mRNA Delivery: Improved Endosomal Escape and Sustained Pharmacology and Safety in Non-human Primates." The American Society of Gene and Cell Therapy. 26 (2018): 1509-1519.
Schoenmaker, Linde, et al. "mRNA-lipid nanoparticle COVID-19 vaccines: Structure and stability." International Journal of Pharmaceutics 601 (2021): 120585-120597.
Song, L.Y., Q.F. Ahkong, Z. Wang Q. Rong, S. Ansell, M.J. Hope, und B. Mui. "Characterization of the inhibitory e¡ect of PEG-lipid conjugates on the intracellular delivery of plasmid and antisense DNA mediated by cationic lipid liposomes." Biochimica et Biophysica Acta 1558 (2001): 1-13.
Szebeni, Janos, et al. "Applying lessons learned from nanomedicines to understand rare hypeersensitivity reactions to mRNA-based SARS-CoV-2 vaccines." Nature Nanotechnology 17 (2022): 337-346.
Tam, P, et al. "Stabilized plasmid-lipid particles for systemic gene therapy." Gene Therapy 7 (2000): 1867-1874.
Tenchov, Rumiana, Janet M. Sasso, und Qiongqiong Angela Zhou. "PEGylated Lipid Nanoparticle Formulations: Immunological Safety." 34 (2023): 941-960.
Urits, Ivan, et al. "A Review of Patisiran (ONPATTRO®) for the Treatment of Polyneuropathy in People with Hereditary Transthyretin Amyloidosi." Neurology and Therapy 9 (2020): 301-315.
Whitehead, Kathryn A., et al. "Degradable lipid nanoparticles with predictable in vivo siRNA delivery activity." Nature Communications 5 (2014): 1-10.
Woodle, Martin C., und Danilo D. Lasic. "Sterically stabilized liposomes." Biochimica et Biophysica Acta, 1113 (1992): 171-199.
Yang, Qi, und Samuel K. Lai. "Anti-PEG immunity: emergence, characteristics, and unaddressed questions." WIREs Nanomed Nanobiotechnology 7 (2015): 655-677.
Zhang, Rui, et al. "Helper lipid structure influences protein adsorption and delivery of lipid nanoparticles to spleen and liver." Biomaterials Science 9 (2021): 1449-14463.
Zhu, Yiran, Liyuan Zhu, Xian Wang, und Hongchuan Jin. "RNA-based therapeutics: an overview and prospectus." Cell Death Dis 13 (2022): 644-658.

### Examples

### 1.1 Example 1: Method for formulation of RNA-LNPs

The RNA-LNPs of the present invention were formulated using a NanoScaler (KNAUER Wissenschaftliche Geräte GmbH, Berlin, Germany) integrated with impingement jet mixing system (Garcia, et al. 2022). The invention involves the formulation of RNA-LNPs through the integration of an organic phase (comprising lipids) and an aqueous phase (containing mRNA payload and dilution buffer) within the NanoScaler.

The organic phase was prepared with a lipid mix consisting of an ionizable lipid, helper lipid, cholesterol and a shielding lipid in defined molar ratio. The aqueous phase consists of diluted mRNA (GFP or Luciferase (Luc)) in 25 mM or 6.25 mM of acetate buffer, depending on the defined formulation maintaining the pH between 4.0 &.5.0. The amine:phosphate ratio (N/P) was maintained at between 6.20 and 5.13. The flow ratio between the organic and aqueous phases was maintained at 1:3, with a flow rate of 4.5 mL/min, comprising 3 mL/min undiluted LNPs and 1.5 mL/min undergoing in-line dilution with PBS.

Following the formulation stage, the LNPs were further diluted in a 10 mM phosphate-buffered saline (PBS) at pH 7.4 to reduce ethanol concentration. Subsequently, purification was achieved using centrifugal concentrators (Vivaspin^{™} 500, Merck KGaA, Darmstadt, Germany) effectively eliminating residual ethanol, thus ensuring the safety and efficacy of the formulation.

The lipid composition of known mRNA based COVID-19 vaccines were used as control (Table 1). The formulation containing the lipids of the invention were prepared analogously with the difference that DPPGₓ or CLGₓ or NLGₓ lipids were used replacing the corresponding lipid in the lipid mix with similar or varying lipid ratios (see following examples).

**Table 1: Description of the mRNA based COVID-19 vaccines which are used as control formulations.**

| **Formulation basis** | **Drug load** | **Buffer** | **Ionizable lipid (%)** | **Helper (%)** | **Chol (%)** | **Shielding (%)** | **N/P ratio** | **Relabeled as** |
|---|---|---|---|---|---|---|---|---|
| Spikevax based | GFP/Luc mRNA | 6.25mM Acetate pH 5.0 | SM-102 (50.0) | DOPE/ DSPC (100) | 38.5 | DMG-PEG (1.5) | 5.13 | **Formulation A** |
| Comirnaty based | GFP/Luc mRNA | 25mM Acetate pH 4.0 | ALC-0315 (46.3) | DOPE /DSPC (9.4) | 42.7 | DMG-PEG (1.6) | 6.17 | **Formulation B** |

### 1.2 Example 2: Synthesis route of cationic/cationizable lipids

The synthesis route for NLG₂ and CLG₂ is depicted below starting from glycidol and 1,2-isopropylideneglycerol. The synthesis proceeds in a stereospecific manner, therefore if needed also distinct stereoisomers can be generated by choice of the enantiomerically pure starting materials in step 1 and 2 (*R*- or *S*-glycidol and *R*- or *S*-1,2-isopropylideneglycerol), as well as step 9 (*R*- or *S*-glycidol). The cationizable NLG₂ was obtained from glycidol and 1,2-isopropylideneglycerol in 11 synthesis steps. The cationic CLG₂ was obtained in 2 additional steps from NLG₂. The synthesis route is applicable to other oligo-containing lipids of Formula (II).

### Synthesis route of NLG₂ and CLG₂

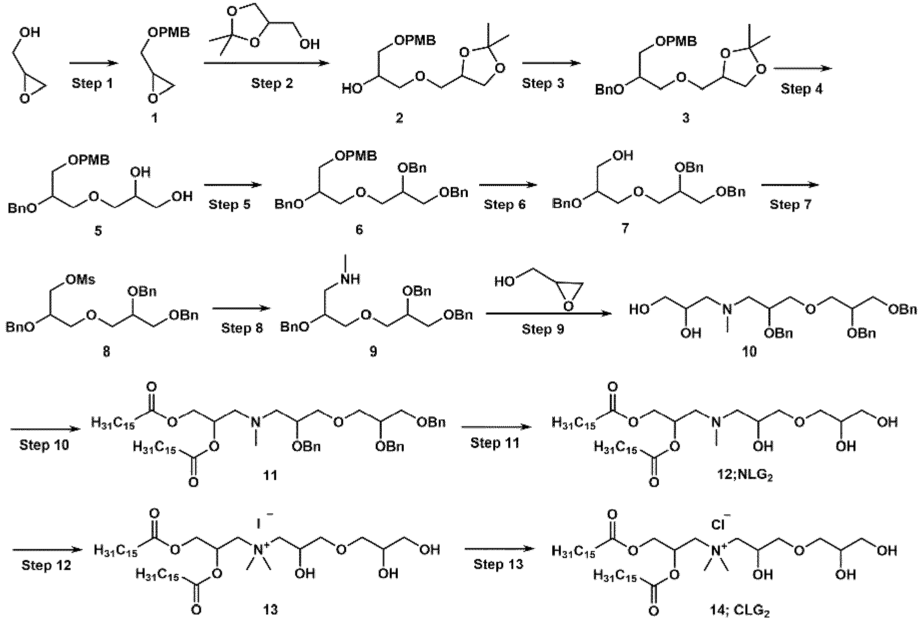

### Step 1:

3.8 kg glycidol are dissolved in 22.8 L NMP, then 8.02 kg PMBCI are added followed by 2.26 kg NaH at 0-5 °C. After 4 hours at RT, the reaction mixture is poured into ice water and extracted with EtOAc. The organic phase is washed with brine, dried over Na₂SO₄ and concentrated under reduced pressure. The isolated residue is purified by silica gel chromatography (n-Heptane/Ethyl acetate) and 8.0 kg of **1** are obtained as colorless oil.

### Step 2+3:

8.0 kg of **1** are dissolved in 64 L DMF, then 8.16 kg 1,2-IPG is added, followed by 6.94 kg tBuOK at 0-5 °C and the resulting reaction mixture stirred for 12 h at RT to obtain **2.** Subsequently, without work up or further purification additional 6.91 kg tBuOK and 12.6 kg benzyl bromide are added at 0-5 °C and the resulting reaction mixture stirred at 4 h at RT. Then, the reaction mixture is poured into ice water and extracted with EtOAc. The organic phase is washed with brine, dried over Na₂SO₄ and concentrated under reduced pressure. 7.0 kg of pure compound **3** is obtained as yellow oil after purification by silica gel chromatography (n-Heptane/Ethyl acetate).

### Step 4:

379 g **3** are dissolved in 2.65 L MeOH and then cooled to 0 °C, where 2 L 1M HCl are slowly added. The reaction mixture is stirred at 25 °C for 4 h. Then, saturated NaHCO₃ solution is added to adjust the mixture to pH = 7 at 0 - 5 °C, followed by extracted with EtOAc. The organic phase is washed brine, dried over Na₂SO₄, filtered, and concentrated under reduced pressure to obtain 310 g of crude **5** as yellow oil.

### Step 5:

310 g of **5** are dissolved in 2.5 L DMF and then cooled to 0 °C, where 369 g tBuOK and 422 g BnBr are added. The reaction mixture is stirred at 25 °C for 4 h, before being poured into ice water followed by extraction with EtOAc. The organic phase is washed brine, dried over Na₂SO₄, filtered and concentrated under reduced pressure. After silica column chromatography (n-heptane/EtOAc) 115 g of pure **6** are obtained as yellow oil.

### Step 6:

331 g of **6** are dissolved in 3 L ACN and 332 ml H₂O and then cooled to 0 °C, where 652 g cerium ammonium nitrate (CAN) are added. The reaction mixture is stirred at 15 °C for 8 h, before being poured into ice water followed by extraction with EtOAc. The organic phase is washed brine, dried over Na₂SO₄, filtered and concentrated under reduced pressure. After silica column chromatography (n-heptane/EtOAc) 168 g of pure **7** are obtained as yellow oil.

### Step 7:

153 g **7** are dissolved in 765 ml DCM, then 71 g triethylamine added, followed by dropwise addition of 50.1 g MsCl. The reaction mixture is stirred at 20 °C for 2 h, before being poured into water followed by extraction with DCM. The organic phase is dried over Na₂SO₄, filtered and concentrated under reduced pressure to obtain 183 g of crude **25** as yellow oil.

### Step 8:

183 g **25** are dissolved in 366 ml EtOH and the resulting solution is added to 1.48 kg of a MeNH₂ solution (33% wt) in EtOH. The reaction mixture is warmed to 55°C and stirred for 48 h, before evaporation to dryness. The residue is taken up in MeOH/toluene and washed with 10 % aqueous Na₂CO₃ solution, before concentration under reduced pressure to obtain 151 g of crude **26** as yellow oil.

### Step 9:

43.6 g **26** are dissolved in 109 ml toluene and then a solution of 9 g glycidol in 109 ml toluene added. The reaction mixture is warmed to 100°C and stirred for 8 h. 3 batches are run in parallel, then combined and evaporated to dryness. The residue is taken up in MTBE and adjusted to pH5 with HCl and then phases separated. The aqueous phase is adjusted to pH8 with saturated NaHCO₃ solution and extracted with ethyl acetate, before concentration of the organic phase under reduced pressure to obtain 161 g of crude **35** as yellow oil.

### Step 10:

161 g **35** are dissolved in 800 ml DCM and then 62.2 g triethylamine, 11.2 g DMAP and 169 g palmitoyl chloride added subsequently. The resulting reaction mixture is stirred at 20°C for 1 h, before pouring into water. After separation the organic phase is concentrated under reduced pressure and then purified by silica column chromatography (n-heptane/EtOAc) to obtain 146 g of pure **36** as yellow oil.

### Step 11:

6 g Pd/C is suspended in 300 ml AcOH and then 67 g of **36** are added and the reactor flushed with 30 psi H₂. The resulting reaction mixture is stirred at 30°C for 24 h. 2 batches are run in parallel and worked up together. After filtration through celite, the filtrate is poured into water and the pH adjusted to 7 with saturated NaHCO₃ solution. After extraction with ethyl acetate, the organic phase is concentrated under reduced pressure. The residue is recrystallized from ACN, followed by purification via silica column chromatography (n-heptane/EtOAc/MeOH) to obtain 38.6 g of pure **NLG₂** as white solid.

### Step 12:

20 g **NLG₂** are suspended in 100 ml ACN and then 7.8 g methyl iodide is added. The resulting reaction mixture is stirred at 40°C for 16 h. After filtration and concentration under reduced pressure 21 g of crude **38** are obtained as white solid.

### Step 13:

21 g **38** are added to a 315 ml 1:1:1 mixture of toluene, 2-propanol and a 10% aqueous NaCl solution. The resulting reaction mixture is stirred at 35°C for 48 h. After isolation, crude product is purified by recrystallization from 2-propanol, followed by multiple triturations with 2-propanol at RT for 1 h to obtain 10 g pure **CLG₂** as white solid. Characterization by ICP-MS confirms CLG₂ as chloride salt.

### 1.3 Example 3: Synthesis of 1,2-dipalmitoyl-sn-glycero-3-phospho-diglycerol and 1,2-dipalmitoyl-sn-glycero-3-phospho-triglycerol

The synthesis route for manufacturing of DPPG₂ and DPPG₃ was described in patent application WO 97/30058 A1 as part of the synthesis of glycerophospholipids of the molecule class of phosphatidyloligoglycerols (PGₓ).

### 1.4 Example 4: Potency of NLG₂ used as ionizable lipid

The cationizable lipid NLG₂ can be used in RNA-LNP formulations to form particles with desirable particle size and PDI but showed low (between 15-25%) mRNA encapsulation efficiency (measured by Ribogreen) (Table 2). This was tested by replacing the cationizable lipid in the Formulation B based LNP formulation (46.3% ALC-0315) as well as in the Formulation A formulation (50% SM-102) with NLG₂. This was not unexpected since NLG₂ does consist of linear and not branched alkyl chains in the lipid tail which are described as necessary for successfully complex mRNA or siRNA. Therefore, encapsulation efficacy can be probably notably improved by using branched fatty acid chains in NLGₓ. The NLG₂-containing RNA-LNPs further possess a pKa value of ~5.2 (Table 2) which is lower than observed for known RNA-LNP (6.2-6.6).

**Table 2: Formulation containing NLG₂ as ionizable lipid replacing ionizable lipid in Formulation A & Formulation B**

| **Formulation basis** | **Ionizable lipid (%)** | **Helper (%)** | **Chol (%)** | **Shielding (%)** | **NIP ratio** | **Size (nm)** | **PDI** | **EE%** | **pka** |
|---|---|---|---|---|---|---|---|---|---|
| Formulation A | NLG₂ (50) | DSPC (10) | 38.5 | DMG-PEG (1.5) | 5.13 | 91 | 0.25 | 24 | 5.1 |
| Formulation B | NLG₂ (463) | DSPC (9.4) | 42.7 | DMG-PEG (1.6) | 6.17 | 113 | 0.22 | 22 | 5.1 |
| Formulation B | NLG₂ (463) | DOPE (9.4) | 42.7 | DMG-PEG (1.6) | 6.17 | 107 | 0.20 | 16 | 5.3 |
| Formulation A | NLG₂ (50) | DOPE (10) | 38.5 | DMG-PEG (1.5) | 5.13 | 99 | 0.19 | 21 | 52 |

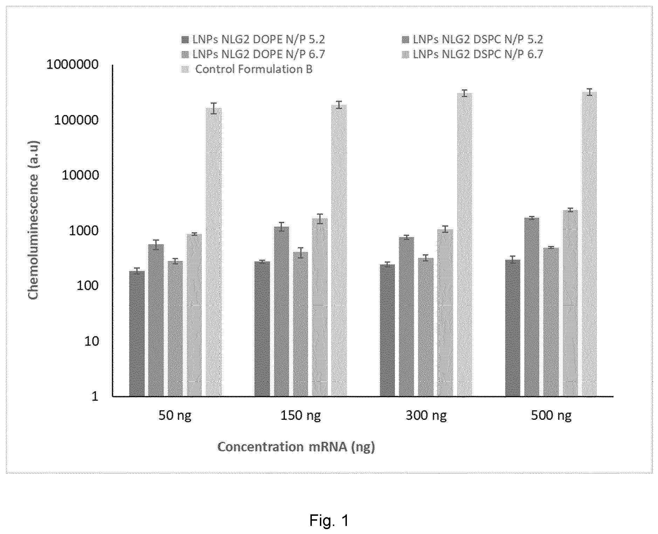

Consequently, the NLG₂ containing LNPs showed only neglectable *in-vitro* transfection efficiency when compared to LNP Formulation B (≤ 1%) (Figure 1). This was the case irrespective of the type of helper lipid (DSPC or DOPE) present in the composition.

Furthermore, increasing the amount of DOPE helper lipid ascendingly from 20% to 30% or varying the N/P ratio in the range from 3.0 to 10.0 resulted in encapsulation efficiencies of less than 25% (data not shown). Additionally, when compared to the LNP Formulation B as control, these NLG₂-containing LNPs had *in vitro* transfection efficiency < 1%.

### 1.5 Example 5: Potency of DPPG₂ lipid as shielding lipid in mRNA-LNP formulation

The effect of three different concentrations of DPPG₂ (2%, 5% and 10%) in RNA-LNP by replacing the PEG-DMG as shielding lipid in the Formulation B was investigated next.

Surprisingly, using DPPG₂ as only shielding lipid resulted in dispersion-stable particles with a size of 130 to 160 nm with a narrow size distribution demonstrated by a PDI ≤ 0.20 (Table 2). Notably, the formulations containing 5% and 10 % anionic DPPG₂, exhibited excellent mRNA encapsulation efficiencies (≥ 80%) (Table 3). This data is indeed remarkable since literature suggests that PEG lipids are mandatory to produce LNPs with an acceptable size range.

**Table 3: Effect of replacing DMG-PEG with DPPG₂ in mRNA based LNPs**

| **Formulation basis** | **mRNA buffer** | **Ionizable lipid (%)** | **Helper (%)** | **Chol (%)** | **Shielding (%)** | **N/P ratio** | **Size (nm)** | **PDI** | **EE (%)** | **pka** | **ZP (mV)** |
|---|---|---|---|---|---|---|---|---|---|---|---|
| Formulation B | 25mM Acetate pH 4 | ALC-0315 (46.3) | DSPC (9.4) | 42.3 | DPPG₂ (2.0) | 6.17 | 156 | 0.14 | >70 | 6.0 | -12 |
| Formulation B | 25mM Acetate pH 4 | ALC-0315 (46.3) | DSPC (9.4) | 39.3 | DPPG₂ (5.0) | 5.13 | 130 | 0.18 | >80 | 6.3 | -20 |
| Formulation B | 25mM Acetate pH 4 | ALC-0315 (46.3) | DSPC (9.4) | 34.3 | DPPG₂ (100) | 6.17 | 140 | 0.2 | >80 | 6.3 | -23 |

It is also observed that with increasing DPPG₂ content in the PEG-free RNA-LNPs the zeta potential of the particles is notably decreasing from -12 mV (2 mol% DPPG₂) to -23 mV (10 mol% DPPG₂) (Table 3). Formulation B RNA-LNPs had a zeta potential of -6.2±0.6 mV. This data suggests that DPPG₂ as a negatively charged phospholipid, has a significant impact on the surface charge indicating that DPPG₂, as well as DPPG₃, is preferentially located in the LNP surface monolayer.

When tested for the stability of mRNA encapsulated in all the DPPG₂ containing RNA-LNPs, it was proved that the mRNA was still intact after 48 hours, among which 10% ratio of DPPG₂ showed comparatively the best performance (Figure 2). The data demonstrated the dispersion stability of DPPG₂-based, PEG free RNA-LNPs.

Interestingly, the presence of serum-containing medium showed a significantly positively influence on the *in vitro* transfection efficiency of DPPG₂-containing LNPs (based on Formulation B) resulting in substantial increase when compared to the PEGylated Formulation B (Figure 3). The difference in transfection can be explained by an improved interaction of the DPPG₂-based RNA-LNPs with the surface of the target cell and/or improved intracellular uptake and/or facilitated release from the endosome. This effect could be majorly attributed due to considerable smaller glycerol headgroups of G₂- and G₃- lipids compared to PEG lipids that are known to sterically hinder such interactions.

The negative influence of the large PEG headgroup on nucleic acid uptake in target cells *in vivo* and *in vitro* has been described (Song, et al. 2001). Interestingly, when investigating the liposome-cell interactions of classical drug-loaded anionic liposomes containing 5% DPPG₂ or 5% DSPE-PEG, respectively, the anionic DPPG₂ liposome showed higher liposome-cell interaction (2-fold in FACS analysis) when compared to its PEGylated counterpart, but, importantly, only in serum-free media, i.e., in the absence of proteins and other serum components. As expected, both anionic liposomal formulations (which had comparable zeta potentials) containing either 5% DPPG₂ or 5% DSPE-PEG2000 showed nearly undetectable cell targeting after protein adsorption (Petrini, et al. 2021).

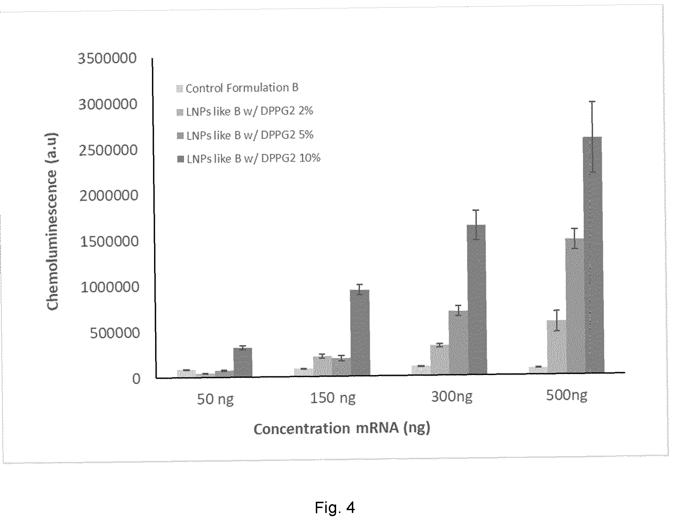

Importantly, all DPPG₂ containing LNPs showed a clear dose-response relationship over the tested range from 50 ng to 500 ng. This is an important prerequisite for the development of a therapeutic RNA-LNP for treatment of patients. This contrasts with the PEGylated Formulation B formulation which showed a constant low transfection efficiency (Figure 4 and Figure 5)

To investigate whether the addition of G₂- or G₃-lipids to the LNP formulation can prevent aggregation with blood components such as erythrocytes after systemic injection, G₂-lipid or G₃-lipid containing and PEGylated RNA-LNPs were added into erythrocyte suspensions. All the G₂- or G₃-lipid modified RNA-LNPs were able to prevent aggregation with erythrocytes.

The influence of DPPG₂ and the effect of PEG lipid as shielding component also plays a significant role on LNP size. This was demonstrated by experiments with selected RNA-LNPs (Table 4). As expected, the size of a PEG-free Formulation B was higher (134 nm) compared to the respective PEG-containing formulation (77 nm) (Table 4). Importantly the size of all three LNPs was in the range of ~77 to 130 nm, which would allow systemic administration in patients, with the DPPG₂ formulation having a particle size of 128 nm and a favourable PDI of 0.15. It is worth mentioning that they had transfection efficiencies of more than 70 %.

The PEG free Formulation A increased in particle size from 116 to 145 nm after 24 hours at 4°C (see Table 4).

**Table 4: LNPs containing DPPG₂ as shielding lipid replacing DMG-PEG and the effect of absence of DMG-PEG on Formulation B based LNP**

| **Stability time** | **Formulation basis** | **Ionizable lipid (%)** | **Helper (%)** | **Chol (%)** | **Shielding (%)** | **Size (nm)** | **PDI** |
|---|---|---|---|---|---|---|---|
| **0 hour** | Formulation B | ALC-0315 (46.3) | DSPC (9.4) | 44.3 | NA | 134 | 0.19 |
| | Formulation A | SM-102 (50.0) | DSPC (100) | 40.0 | NA | 116 | 0.17 |
| | Formulation B | ALC-0315 (46.3) | DSPC (9.4) | 42.7 | DMG-PEG (1.6) | 77 | 0.19 |
| | - | ALC-0315 (46.3) | DSPC (9.4) | 34.3 | DPPG₂ (100) | 128 | 0.15 |
| **24 hours** | Formulation B | ALC-0315 (46.3) | DSPC (9.4) | 44.3 | NA | 134 | 0.17 |
| | Formulation A | SM-102 (50.0) | DSPC (100) | 40.0 | NA | 145 | 0.19 |

To further assess the shelf-life stability of different RNA-LNPs, we conducted a comparison between two formulations containing either DPPG₂ or DMG-PEG as shielding lipid (Table 5). The DPPG₂-containing RNA-LNP had a lipid composition of ALC-0315/DSPC/Chol/DPPG₂ 46.3:9.4:34.3:10 (mol/mol) while the PEGylated RNA-LNP were composed of ALC-0315/DSPC/Chol/DMG-PEG 46.3:9.4:42.7:1.6 (mol/mol). As buffer, 25 mM acetate pH 4.0 was used as buffer for both formulations. Remarkably, both formulations demonstrated sustained stability, maintaining constant particle size even after one week of storage at 2-8°C. However, when stored at-20°C, both formulations exhibited a comparable increase in particle size. Apparently, the presence of sucrose as model cryoprotectant did not have an influence on the increase in size. The encapsulation efficiency for the GFP mRNA remained good for all formulations as analysed by agarose gel electrophoresis (data not shown).

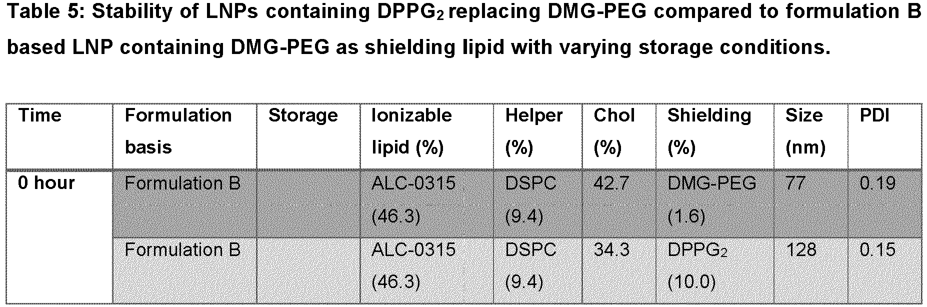

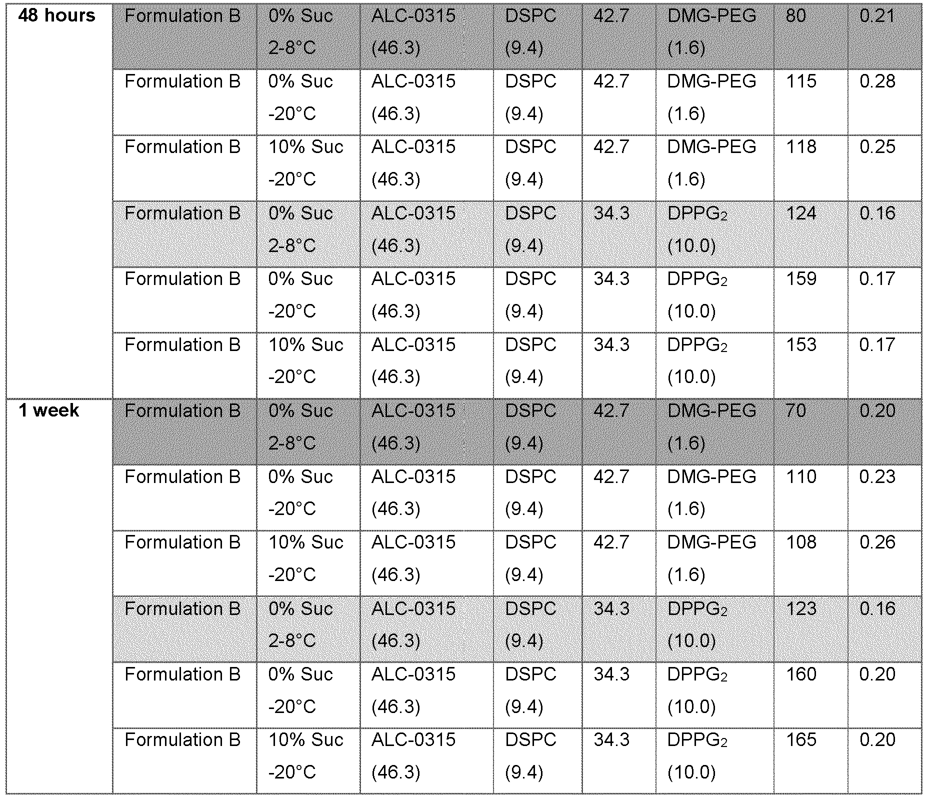

### 1.6 Example 6: Potency of DPPG₃ lipid as shielding lipid in mRNA-LNP formulation

In a next step, ability of DPPG₃ to replace PEG-DMG as shielding lipid in RNA-LNP was investigated. The lipid compositions and characterization results of the formulations are shown in Table 6.

DPPG₃ (5 or 10 mol%) was successfully used in RNA-LNP to replace the shielding lipid DMG-PEG in both Formulation A and Formulation B like LNPs. RNA-LNPs with well-defined size, a narrow size distribution with a PDI <0.2), and a good mRNA encapsulation efficiency (70-83%) were produced.

**Table 6: Incorporation of 5% and 10% DPPG₃ into the Formulation A & B based formulations**

| **Formulation basis** | **Ionizable lipid (%)** | **Helper (%)** | **Chol (%)** | **Shielding (%)** | **NIP ratio** | **Size (nm)** | **PDI** | **ZP (mV)** | **EE (%)** |
|---|---|---|---|---|---|---|---|---|---|
| Formulation B (control) | ALC-0315 (463) | DSPC (100) | 42.7 | DMG-PEG (1.6) | 6.17 | 84 | 0.18 | -4 | 72 |
| Formulation B | ALC-0315 (46.3) | DSPC (9.4) | 39.3 | DPPG₃ (5.0) | 6.17 | 177 | 0.16 | -18 | 83 |
| Formulation B | ALC-0315 (46.3) | DSPC (9.4) | 34.3 | DPPG₃ (100) | 6.17 | 160 | 0.15 | -24 | 82 |
| Formulation A (TRIS) | SM-102 (50) | DSPC (100) | 35.0 | DPPG₃ (5.0) | 5.13 | 208 | 0.14 | -21 | 79 |
| Formulation A (TRIS) | SM-102 (50.0) | DSPC (100) | 300 | DPPG₃ (100) | 5.13 | 185 | 0.09 | -16 | 75 |
| Formulation A (PBS) | SM-102 (50.0) | DSPC (100) | 35.0 | DPPG₃ (5.0) | 5.13 | 164 | 0.15 | -15 | 83 |
| Formulation A (PBS) | SM-102 (50.0) | DSPC (100) | 300 | DPPG₃ (100) | 5.13 | 143 | 0.14 | -18 | 70 |

Also, the *in vitro* transfection efficiency (in serum-containing medium) of DPPG₃-containing RNA-LNPs was increased compared to the PEGylated Formulation B LNP (Figure 6). A striking increase in transfection efficiency was observed especially for the PEG-free Formulation A RNA-LNPs applying SM-102 and DPPG₃ as cationizable and shielding lipid, respectively (Figure 6), respectively.

**Table 7: Effect on zeta potential by replacing DPPG₃ as shielding lipid with Formulation A & B based LNPs**

| **Formulation basis** | **mRNA buffer** | **Zeta Potential (mV)** |
|---|---|---|
| Formulation B (control) | 25 mM Acetate pH 4 | -4.3 |
| Formulation B based DPPG₃ (5%) LNPs | 25 mM Acetate pH 4 | -19.6 |
| Formulation B DPPG₃ (10%) LNPs | 25 mM Acetate pH 4 | -23.8 |
| Formulation A based DPPG₃ (5%) LNPs | TRIS | -27.5 |
| Formulation A based DPPG₃ (10%) LNPs | TRIS | -28.5 |
| Formulation A based DPPG₃ (5%) LNPs | PBS | -15.2 |
| Formulation A based DPPG₃ (10%) LNPs | PBS | -18.5 |

### 1.7 Example 7: Effect of cationic CLG₂ lipid in mRNA-LNPs

The possibility of increasing the zeta potential of PEGylated RNA-LNPs based on Formulation A and B while achieving a well-controlled particle size and maintaining a satisfying transfection efficiency was investigated next. For this purpose, the new lipid CLG₂, a cationic member of the G₂ lipid family, which carries a permanent positive charge was used.

Initial experiments were for testing whether the cationic CLG₂ could replace the neutral charged helper lipid DSPC in Formulation A & B. Final lipid compositions and characterization results are shown in Table 8. The PDI of the CLG₂-containing PEGylated RNA-LNPs (Table 8) were comparable to the control Formulation B (Table 6). However, particle size was larger for the former. The encapsulation efficiencies were > 80% for the CLG₂-containing PEGylated RNA-LNPs. The pKa values (as measured by TNS assay) were in the range of 6.2 to 6.3 for all three formulations. It is important to mention that all formulations had still a negative zeta potential which is favourable for *in vivo* administration but was slightly higher than for Formulation B (Table 6). However, since PEG is known to successfully shield the charge of membrane forming lipids, the effect of CLG₂ on the surface charge might be underrepresented by the zeta potential results.

So, surprisingly, the cationic CLG₂ can replace the neutral DSPC as helper lipid in PEGylated RNA-LNP formulations, thereby modulating the zeta potential and potentially also influence the protein corona of the resulting LNPs *in vivo* after systemic administration.

Importantly, the particle size of PEGylated CLG₂-containg RNA-LNPs was affected by the structure of the cationizable lipid and the amount of CLG₂ used (Table 8). Particle size was 170 nm (PDI 0.19) and 218 nm (PDI 0.19) for RNA-LNP using SM-102 (Formulation A like) and ALC-0315 (Formulation B like) for RNA-LNP using ~10 mol% CLG₂ as helper lipid, respectively. When increasing CLG₂ content from 10 mol% to 20 mol% in RNA-LNP using 50 mol% SM-102 (Formulation A like) particle size was decreased from 170 nm (PDI 0.19) to 148 nm (PDI 0.17), respectively. Thus, it can be assumed that smaller LNPs can be generated by incorporation of higher levels of CLG₂ even in the total absence of PEG-lipid. The mRNA in the CLG₂ formulations was not degraded after 48 hours (data not shown).

**Table 8: Potency of CLG₂ as helper lipid**

| **Formulation basis** | **mRNA buffer** | **Ionizable lipid (%)** | **Helper (%)** | **Chol (%)** | **Shielding (%)** | **NIP ratio** | **Size (nm)** | **PDI** | **ZP (mV)** | **pKa** | **EE (%)** |
|---|---|---|---|---|---|---|---|---|---|---|---|
| Formulation A | 6.25mM Acetate pH 5.0 | SM-102 (500) | CLG₂ (100) | 38.5 | DMG-PEG (1.5) | 5.13 | 170 | 0.19 | -1.8 | 6.3 | >80 |
| Formulation A | 6.25mM Acetate pH 5.0 | SM-102 (50.0) | CLG₂ (20.0) | 38.5 | DMG-PEG (1.5) | 5.13 | 148 | 0.17 | -1.2 | 6.3 | >80 |
| Formulation B | 25m M Acetate pH 4.0 | ALC-0315 (46.3) | CLG₂ (9.4) | 42.7 | DMG-PEG (1.6) | 6.17 | 218 | 0.19 | -1.0 | 6.5 | >60 |

In the presence of a serum-containing medium, the *in vitro* transfection efficiency of PEGylated RNA-LNPs containing CLG₂ as a helper lipid exhibited a marked improvement compared to the PEGylated LNPs found in Formulation B (Figure 7).

**Table 9: Potency of CLG₂ to replace the ionizable lipid in Formulation A based LNPs**

| **Formulation basis** | **Ionizable lipid (%)** | **Helper (%)** | **Chol (%)** | **Shielding (%)** | **NIP ratio** | **Size (nm)** | **PDI** |
|---|---|---|---|---|---|---|---|
| Formulation A | CLG₂ (40.0) | DSPC (20.0) | 38.50 | DMG-PEG (1.5) | 5.13 | 295 | 0.30 |
| Formulation A | CLG₂ (50.0) | DSPC (100) | 38.50 | DMG-PEG (1.5) | 5.13 | 328 | 0.29 |

As expected, CLG₂ containing Formulations A & B could only form micro-particles with wide size distribution (>0.25) when CLG₂ having linear acyl chains was used to replace the respective ionizable lipid with branched alkyl chains in a LNP formulation (Table 9).

Interestingly, CLG₂ could also not stabilize RNA-LNPs in the absence of PEG-DMG or DPPG₂ (see Table 10) resulting in unfavourable particle sizes and PDIs even after buffer exchange.

**Table 10: Potency of CLG₂ as shielding lipid by replacing DMG-PEG in mRNA-LNPs**

| **Formulation basis** | **Ionizable lipid (%)** | **Helper (%)** | **Chol (%)** | **Shielding (%)** | **NIP ratio** | **Size (nm)** | **PDI** |
|---|---|---|---|---|---|---|---|
| **Formulation B** | ALC-0315 (46.3) | DSPC (9.4) | 42.7 | CLG₂ (2) | 6.17 | 298 | 0.19 |
| **Formulation B** | ALC-0315 (46.3) | DSPC (9.4) | 39.3 | CLG₂ (5) | 6.17 | 363 | 0.31 |
| **Formulation B** | ALC-0315 (46.3) | DSPC (9.4) | 34.3 | CLG₂ (10) | 6.17 | 318 | 0.22 |
| **Formulation A** | SM-102 (50.0) | DSPC (10.0) | 38.0 | CLG₂ (2) | 5.13 | 551 | 0.25 |
| **Formulation A** | SM-102 (50.0) | DSPC (10.0) | 35.0 | CLG₂ (5) | 5.13 | >1200 | 0.30 |
| **Formulation A** | SM-102 (50.0) | DSPC (10.0) | 30.0 | CLG₂ (10) | 5.13 | 638 | 0.30 |
| **Formulation A** | SM-102 (50.0) | DSPC (10.0) | 38.0 | CLG₂ (2) | 5.13 | 388 | 0.07 |
| **Formulation A** | SM-102 (50.0) | DSPC (10.0) | 35.0 | CLG₂ (5) | 5.13 | 489 | 0.17 |
| **Formulation A** | SM-102 (50.0) | DSPC (10.0) | 30.0 | CLG₂ (10) | 5.13 | 486 | 009 |

### 1.8 Example 8: Potency of CLG₂ (cationic lipids) & DPPG₂ as helper lipid in mRNA-LNPs

In a next step we combined two members of the G₂-lipid family, the anionic DPPG₂ and the cationic CLG₂ in RNA-LNPs to replace the helper lipid DSPC and PEGylated stabilizer lipids, respectively. Table 11 depicts the used lipid compositions and characterization results of the prepared formulations. Both RNA-LNP formulations had good pKa values and good encapsulation efficiencies. Further, the potential of CLG₂ to modulate the zeta potential of the RNA-LNP was again demonstrated. This is clearly shown by comparison of the RNA-LNPs containing 9.4% CLG₂ and 10% DPPG₂ (zeta potential: -6.5±0.4 mV; Table 11), or RNA-LNPs containing 9.4% CLG₂ and 1.6% DMG-PEG (-1.0±0.5 mV; Table 8), versus RNA-LNPs containing 0% CLG₂, 9.4% DSPC and 10% DPPG₂ (-23±1.8 mV; Table 3).

The LNP formulation, which contains nearly equimolar amounts of the negatively charged DPPG₂ (10%) and the positively charged CLG₂ (9.4%) has similar zeta potential (-6.5±0.4 mV) as compared to the "standard" LNP Formulation B which contains 9.4% of the zwitterionic DSPC (zeta potential -6.2±0.4 V).

Remarkably, RNA-LNP consisting of CLG₂ and DPPG₂ showed up to 10-fold higher *in vitro* transfection efficiency than the PEGylated RNA-LNPs (see Figure 8).

Thus, by combining cationic and anionic members of the G₂- and G₃-lipid family it is possible to design and produce PEG free mRNA LNPs which share several features (including the almost same zeta potential) with the approved COVID-19 vaccines (Formulation B and Formulation A) and the approved siRNA LNP Onpattro^{®} (Urits, et al. 2020).

CLG₂ could also facilitate charge (zeta potential) modulation for LNP formulations in the presence of a shielding agent, e.g., a PEGylated lipid or an anionic member of the G₂- or G₃-lipid family.

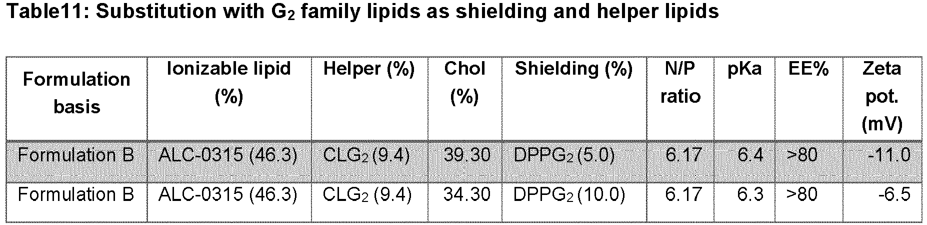

Therefore, the presence of G₂- or G₃-lipids in RNA LNP formulations may have the potential to ensure systemic stability without the loss of transfection activity by PEGylation.

Using DPPG₂ or DPPG₃ as a replacement for PEG-lipids in LNPs would offer a promising solution for the application of RNA-LNPs for *in vivo* applications. Moreover, cationic G₂- or G₃-lipids which also appears to be LNP compatible, could serve as a cationic component to modulate the zeta potential, if necessary.

### 1.9 Example 9: Multi-layer LNPs

### Example 9.1: Material and methods

The multilayer LNPs described in this example had been prepared by DC and batches were characterized by DLS (hydrodynamic diameter and PDI), time-resolved fluorescence measurements (encapsulation efficacy, EE) and Cryo-TEM (morphology) as published (Koehler, et al. 2023). Lipid-related decomposition products (fatty acids) were quantified by HPTLC and calcein release kinetics were measured by fluorescence spectrophotometry, respectively.

Materials: Hydrogenated egg phosphatidylcholine (HEPC; ≥98%), egg phosphatidylcholine (EPC; ≥96%), 1,2-dipalmitoyl-sn-glycero-3-phosphocholine (DPPC; ≥99%), 1,2-distearoyl-sn-glycero-3-phosphocholine (DSPC; ≥99%), and N-(Carbonyl-methoxypolyethylene glycol-2000)-1,2-distearoyl-sn-glycero-3-phosphoethanolamine, sodium salt (DSPE-PEG2000; ≥98%) were kindly provided by Lipoid GmbH (Ludwigshafen, Germany). Calcein (product number: C0875; batch number: SHBM5832) and cholesterol (≥99%) were obtained from Sigma-Aldrich (St. Louis, MO, USA). Tris(hydroxymethyl)aminomethane (Tris; ≥99.9%) and ethylenediaminetetraacetic acid (EDTA; ≥99%) were obtained from Carl Roth GmbH (Karlsruhe, Germany). All other chemicals were obtained from Carl Roth GmbH (Karlsruhe, Germany) and were of analytical grade.

In-vial lipid film preparation: Lipid films were prepared using a modified thin-film hydration method for each lipid mixture. The dry lipids were dissolved in a chloroform/methanol (2:1, vol/vol) mixture to obtain a stock solution of 200 mM lipid concentration. Aliquots from this stock solution were pipetted directly into 2 mL conical screw cap vials (Sarstedt AG & Co. KG, Nümbrecht, Germany, Type 72.693.005). The organic solvents were evaporated at 38-41°C in a vacuum centrifuge (RVC 2-18, Martin Christ Gefriertrockungsanlagen GmbH, Osterode, Germany) with an attached vacuum pump (MZ 2C, Vacuubrand GmbH + Co KG, Wertheim, Germany) for 4-8 h until a dry lipid film was formed. To ensure complete evaporation of the organic solvent, the lipid films were dried in a vacuum desiccator with a high-vacuum pump (RD 4, Vacuubrand GmbH + Co KG, Wertheim, Germany) for at least 2 h.

Dual centrifugation homogenization: samples were prepared by dual centrifugation (DC) using a ZentriMix 380 R (Andreas Hettich GmbH & Co. KG, Tuttlingen, Germany). All samples for the lipid mixture screenings were prepared in 2 mL conical screw cap vials, each with 600 mg zirconium oxide beads with a diameter of 1.4-1.6 mm (SiLibeads^{®} Type ZY-S, Sigmund Lindner GmbH, Germany), the lipid film, and calcein buffer (60 mM, 10 mM TRIS, 0.5 mM EDTA, pH 8.5) or only TRIS buffer as the aqueous phase. Liposome or LNP preparation by DC was also conducted with lipid mixtures without prior lipid film preparation. Samples for preparation by DC without lipid film preparation were prepared by weighting the dry lipid components, the zirconium oxide beads, and the aqueous phase directly into the vial. The standard settings for liposome preparation in DC were 2350 rpm, 30 min, and 20 °C or 40 °C when using DPPG₂ as part of the lipid blend. The resulting vesicular phospholipid gel (VPG) was diluted to a suspension with TRIS buffer (10 mM TRIS buffer containing 0.5 mM EDTA, pH 8.5) in the ratio 2:1 (vol/vol) by DC (1500 rpm, 2 min, 20 °C). For all lipid mixtures and lipid concentrations, a batch size of 100 mg VPG was prepared to standardize the influence of the beads and wetting of the vial surface. According to the lipid concentration used (2.5-85%), 2.5-85 mg lipid mixture and corresponding to 97.5-15 µL of aqueous buffer were used for the preparation of each VPG.

Size and size distribution: Dynamic light scattering (DLS, Zetasizer Nano-ZS, Malvern Instruments, Worcestershire, UK) was used to determine the intensity-weighted hydrodynamic diameter (Z-average) and polydispersity index (PDI) of the DC-produced batches. Dispersions were diluted with TRIS buffer (viscosity: 0.8749 mPa*s; refractive index: 1.330). The attenuator was set automatically to reach a count rate of about 150-250 kcps. The instrument evaluated the backscattering intensity from a 4 mL polystyrene cuvette with a layer thickness of 10 mm (Sarstedt AG & Co. KG, Nümbrecht, Germany) at an angle of 173°. Three measurements with an automatically selected number of scans (10-17 scans, 10 s per scan) were taken for each sample.

Time-resolved fluorescence measurements (entrapping efficiencies): The batches after preparation and redispersion in a DC was diluted 1:5000 with TRIS buffer (4 µL liposome suspension + 1996 µL TRIS buffer; thereof 200 µL + 1800 µL TRIS buffer). Time-resolved fluorescence measurements were conducted at the fluorescence lifetime spectrometer FluoTime 100 (PicoQuant, Berlin, Germany). The excitation source was a modular laser diode (LDH-P-C-470) with a wavelength of 470 nm (± 10 nm), pulsed at a repetition rate of 20 MHz. The laser was operated with the laser driver PDL 800-D (PicoQuant, Berlin, Germany). Decay curves were recorded at 515 nm wavelength by using time-correlated single photon counting (TCSPC). For each sample, the measurement conditions were set to a peak count of at least 10,000 photons by varying Phar the transmission (0.1-1%) and the measuring period (20-50 s). Fluorescence decay curves were fitted with FluoFit software (PicoQuant, Berlin, Germany) by a biexponential function.

Cryo-EM: The samples for the cryo-EM analysis were prepared by DC with plain TRIS buffer and diluted. Samples were equilibrated at 25 °C and a high relative humidity in a climate chamber. A total of 1 µL of each sample was deposited on a carbon-sputtered copper grid covered with a perforated polymer film. Then, excess liquid was removed by blotting with filter paper, leaving a thin film of the solution on the grid. The specimen was immediately vitrified in liquid ethane and transferred to the microscope, continuously kept below -160 °C, and protected against atmospheric conditions. Analyses were performed with a Zeiss Libra 120 transmission electron microscope (Carl Zeiss AG, Oberkochen, Germany) operating at 80 kV in zero-loss bright-field mode. Digital images were recorded under lowdose conditions with a BioVision Pro-SM Slow Scan CCD camera (Proscan Elektronische Systeme GmbH, Scheuring, Germany).

Statistical Analysis: The data are presented as mean ± standard deviation (SD) of three independent experiments. DSC experiments are illustrated as a combined thermogram of single scans for each sample.

### Example 9.2: Morphology of DPPG₂-based multilayer LNPs

Different batches of the lipid mix with composition DPPC/DSPC/DPPG2 50:20:30 (mol/mol) differing in lipid mix concentration in aqueous media were prepared by DC. At low concentrations (< 60 wt%), the DC homogenization resulted in classical unilamellar liposomes. In contrast, high concentrations (≥ 60 wt%) of the lipid mixture led surprisingly to unusually tightly packed particles (Figure 9C) with beneficial properties for drug delivery systems. For example, homogenization with 60 wt% yielded particles in the preferred size range for various *in vivo* applications routes (~150 nm) with a very narrow size distribution (PDI < 0.05) and a high encapsulation efficacy (EE ~30%) for passive loading procedures (example 9.3, Figure 10). This new type of LNP (termed multilayer LNPs thereafter) have an inner core resembling inverse micellar structures (hexagonal structures) which is covered by multiple membranes. Furthermore, it can be observed that the spacing between these bilayers decreases as the curvature increases (Figure 9C). As defined for LNPs, the new particles have virtually no aqueous internal volume.

The morphology of DPPG₂-based multilayer LNPs was insofar surprising as other lipid mixtures such as hydrogenated egg PC (HEPC)/cholesterol clearly result in small unilamellar vesicles according to (Koehler, et al. 2023) in which the multiple bilayers are equidistant and there is also a tiny aqueous inner volume (Figure 9A). Since this is also the case for using pure DPPC, the formation of the newly discovered LNPs bases is the result of the DPPG₂/₃ as part of the lipid blend. In case of PEGylated lipids present in HEPC/cholesterol mixtures, the population of multilamellar liposomes was clearly smaller for the PEG-containing samples, and the liposomes tended to contain fewer and less densely packed lamellae than seen in HEPC/cholesterol mixtures (Figure 9C) (Koehler, et al. 2023). PEG-containing lipid mixtures are therefore not prone to form multilayer LNPs.

The described difference in morphology is most probably due to a so far unknown interaction between the diglycerol headgroups of DPPG₂ due to polar and protic interaction (hydrogen bonding). That such a behaviour hasn't been observed for PEGylated lipids and can be explained by the fact that the ethylene groups can only interact with water, but not with both, water as well as other PEGylated lipids. In contrast to PEG, diglycerols have hydroxyl-groups that can interact via hydrogen-bonding with the hydroxyl-groups of other DPPG₂ molecules being in the same or the opposing, neighbour membranes. Since membranes containing DPPG₂ or DPPG₃ with packing parameters of >1 tend to form rather planar membranes, it was surprising that the newly discovered cooperative effects between the DPPG₂-containing membranes forces the membranes to form perfectly round particles, an effect which makes those particles more stable against membrane interactions with serum proteins (refer to example 9.5).

Moreover, the absence of an inner water core can also be attributed to this particular property of DPPG₂ or DPPG₃ to displace water from the membranes' interstitial space through interaction with other oligoglycerol-containing lipids, especially at stronger curvature (Figure 9C). The reduced amount of water between the membranes is expected to result in reduced hydrolysis either of encapsulated compounds (e.g., nucleic acid, small molecules) or of the multilayer LNP-forming phospholipid excipients (refer to example 9.4).

Another surprising advantage was that the above described LNPs could be produced while omitting organic solvents in the manufacturing process by using the in-vial homogenization technique (DC, dual centrifugation).

### Example 9.3: Effect of lipid concentration in DC homogenization on particle size and encapsulation efficiency

Figure 10 demonstrates the effect of lipid concentration on the hydrodynamic diameter and polydispersity index (PDI) of the formed particles from DPPC/DSPC/DPPG₂ 50:20:30 (mol/mol) lipid mixtures. Moreover, the encapsulation efficacy (EE) of the hydrophilic small molecule, fluorescent dye, and model drug calcein is depicted which was passively encapsulated during the manufacturing process. The high EE allows the efficient and economic formulation of nucleic acids and active pharmaceutical ingredients for drug delivery purposes.

### Example 9.4: Chemical stability of lipids in DPPG₂-based multilayer LNPs

Here the chemical stability of the lipids in the preparation of multilayer LNPs using the DC homogenization process described in example 9.1 was investigated. Decomposition of phospholipids in aqueous solutions by hydrolysis usually leads to formation of a fatty acid (FA) and the corresponding lysolipid. Either DPPC/DSPC/DPPG₂ 50:20:30 (mol/mol) or pure DPPG₂ was used, respectively. DC homogenization was performed with a lipid concentration of either 20 wt% or 60 wt%, aiming small unilamellar liposomes or multilayer LNPs, respectively. The total FA content in each preparation was measured after preparation and the results are depicted in Figure 11. Surprisingly, the DPPC/DSPC/DPPG₂ mixture was notably more stable as multilayer LNP (60 wt%) than in small unilamellar liposomes (20 wt%). The same was demonstrated for pure DPPG₂ particles. Moreover, pure DPPG₂ particles are more stable than DPPC/DSPC/DPPG₂ mixtures independent from their morphology. Furthermore, the multilayer LNP are surprisingly stable, even after their dilution in an excess of water, meaning that their formation seems to be not reversal (including morphology, data not shown).

Another important finding was that hydrolysis of phospholipids can be reduced by adding poly-alcohols (e.g., sucrose, trehalose, mannitol, glycerol) during DC homogenization of a DPPC/DSPC/DPPG₂ 50:20:30 (mol/mol) lipid mixture (Figure 12).

In conclusion, a particular advantage of these multilayer LNPs made with higher lipid concentrations by DC is their enhanced stability with respect to lipid hydrolysis. This might allow storage of DPPG₂ containing LNPs even at room temperature, 2-8°C or by simply freezing the dispersion. Thus, it was shown that the storage of those LNPs in aqueous buffer at 4 °C and frozen at -20 °C can be performed without severe hydrolysis. The reduced hydrolysis rate in multilayer LNPs can be explained by several factors. The high lipid concentration during preparation leads to a shortage of water while the interdigitation of the oligoglycerol headgroups due to polar and protic interaction (hydrogen bonding) are probably displacing water molecules from the near proximity of the phospholipid ester bonds necessary for hydrolysis.

### Example 9.5: Temperature-dependent release of calcein from DPPG₂-based multilayer LNPs

The effect of the particle morphology of DPPC/DSPC/DPPG₂ 50:20:30 (mol/mol) lipid mixtures on the temperature-dependent calcein release kinetics was investigated in either TRIS buffer or in presence of the human serum albumin (HSA) protein. Particles were prepared by DC homogenization as described in example 9.1 and a lipid concentration of either 40 wt% or 60 wt% to obtain small unilamellar liposomes or multilayer LNPs, respectively. The results are shown in Figure 13. The classical thermosensitive liposomes (TSL) demonstrated some unwanted calcein leakage already at body temperature (37°C, Figure 13, left), an effect that is known for virtually all other known TSL formulations. Importantly, multilayer LNPs formed by the same lipid composition showed virtually no release (37°C, Figure 13, right). At temperatures approaching the known solid gel to liquid disordered phase transition temperature (Tₘ) of the lipid mixture, small unilamellar liposomes showed a faster calcein release than the multilayer LNPs (42°C, Figure 13). Interestingly, multilayer LNPs are less prone to the destabilizing effect of serum proteins which are known to increase leakage rate independent from the temperature (Figure 13, bottom). This can be explained by the protective effect of the outermost bilayer of the multilayer LNPs that prevents the access of the proteins to the inner bilayers.

### 1.10 Example 10: Formation of NLG₂-based PEGylated RNA-LNPs with DC

In this example, the LNP ethanol injection preparation process described in example 1 above was adapted for the dual centrifugation process. The new process (Figure 14) was tested with the lipid mixtures NLG₂/Chol/DSPC/DSPE-PEG2000 46.3:42.7.11-x:x (mol/mol) (x = 0, 0.6, 1.6, 2.6, and 5 mol%) and was performed without organic solvents. The lipid mixture was mixed with 300 mM citric acid pH 4 (with or without siRNA), and beads at the desired lipid concentration. After in-vial DC homogenization (2350 rpm, 10 min) a vesicular phospholipid gel (VPG) is formed. By addition of a neutralization buffer (e.g., 270 mM sucrose, 30 mM HEPES, pH 7.4 [SH-buffer], with or without siRNA) and subsequent DC (1000 rpm, 2 min) LNPs are formed.

Figure 15 summarizes the effect of the DSPE-PEG2000 content on the particle size (expressed as z average) and particle size distribution (expressed as PDI) of preparations produced either with 20 wt% or 50 wt% lipid concentration. All batches were produced without addition of siRNA. The preparation of LNPs at high lipid concentrations led to formation of stable structures which do not tend to aggregate, independently if there is a PEG-lipid part of the lipid mixture or not (Figure 15, right). Thus, the preparation process can be used to prepare nucleic or drug containing LNPs in only one step and without the need of organic solvents. In contrast, performing the DC at lower lipid concentration, small liposomal vesicles have been formed which, since they are not completely filled with membranes and therefore stabilized, are able to aggregate when the positively charge has been neutralized. If this is the case, the amount of PEG has a clear influence on the final LNP-size (Figure 15, left). This strategy can be used to fine tune the size of the resulting LNPs.

Finally, different batches of RNA-LNPs were produced using the lipid mixture NLG₂/cholesterol/DSPC/DSPE-PEG2000 46.3/42.7/8.4/2.6 (mol/mol) and the process as described above (Figure 14). The siRNA (dissolved in RNAse-free water) was either added before DC-homogenization in a 50 mM citric acid pH 4 buffer (loading 1) or after the DC-homogenization in the SH-buffer (loading 2), respectively. Loading 1 and loading 2 resulted in homogeneous particle distributions with a z average of 178 nm (PDI 0.184) and 154 nm (PDI 0.165), respectively. The siRNA encapsulation efficacy (Ribogreen assay) was high for loading 1 (70%) but weak for loading 2 (16%). Both batches were stored at 2-8°C and z average and PDI was measured again after 1 day, 5 days, and 14 days, respectively. DLS results for RNA-LNP (loading 1) are 178 nm (0.184), 165 nm (0.188), and 167 nm (0.152), respectively. DLS results for RNA-LNP (loading 2) are 154 nm (0.165), 144 nm (0.140), and 146 nm (0.136), respectively. Therefore, particles were stable and did not show signs of aggregation for at least 14 days when stored at 2-8°C.

The present application also discloses the following items:
Item 1. A composition comprising:
   a) at least one oligoglycerol-containing lipid of Formula (I), 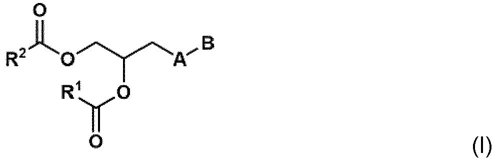
      wherein A = O-P(-O⁻)(=O)-O; or NR³ ; or N⁺R⁴R⁵ ;
      R¹ and R² each independently are a hydrocarbon group having 12 to 24 C-atoms;
      R³, R⁴ and R⁵ each independently are hydrogen or a hydrocarbon group having 1-5 C-atoms; and
      B = a linear or branched oligoglycerol group having from 2 to 20 glycerol units; and
   b) at least one further lipid selected from
      (i) at least one cationic or cationizable lipid;
      (ii) at least one structural lipid;
      (iii) at least one helper lipid; and/or
      (iv) at least one PEG lipid.
Item 2. The composition of item 1 comprising:
   a) at least one oligoglycerol-containing lipid of Formula (I), 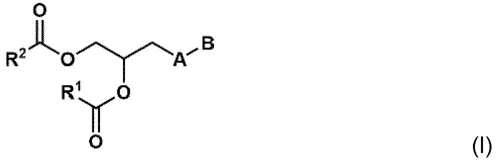
      wherein A = O-P(-O⁻)(=O)-O; or NR³ ; or N⁺R⁴R⁵ ;
      R¹ and R² each independently are a hydrocarbon group having 12 to 24 C-atoms;
      R³, R⁴ and R⁵ each independently are hydrogen or a hydrocarbon group having 1-5 C-atoms; and
      B = a linear or branched oligoglycerol group having from 2 to 20 glycerol units; and
   b)(i) at least one cationic or cationizable lipid; and
   b)(ii)at least one structural lipid.
Item 3. The composition of item 1 comprising:
   a) at least one oligoglycerol-containing lipid of Formula (I), 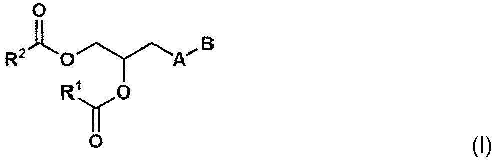
      wherein A = O-P(-O⁻)(=O)-O; or NR³ ; or N⁺R⁴R⁵ ;
      R¹ and R² each independently are a hydrocarbon group having 12 to 24 C-atoms;
      R³, R⁴ and R⁵ each independently are hydrogen or a hydrocarbon group having 1-5 C-atoms; and
      B = a linear or branched oligoglycerol group having from 2 to 20 glycerol units; and
   b)(i) at least one cationic or cationizable lipid;
   b)(ii)at least one structural lipid; and
   b)(iii) at least one helper lipid.
Item 4. The composition of item 1 comprising:
   a) at least one oligoglycerol-containing lipid of Formula (I), 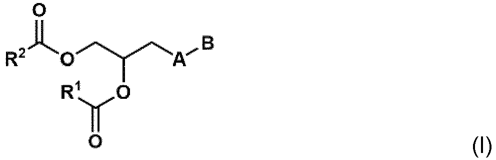
      wherein A = O-P(-O⁻)(=O)-O; or NR³ ; or N⁺R⁴R⁵ ;
      R¹ and R² each independently are a hydrocarbon group having 12 to 24 C-atoms;
      R³, R⁴ and R⁵ each independently are hydrogen or a hydrocarbon group having 1-5 C-atoms; and
      B = a linear or branched oligoglycerol group having from 2 to 20 glycerol units; and
   b)(i) at least one cationic or cationizable lipid;
   b)(ii)at least one structural lipid; and
   b)(iv) at least one PEG-lipid.
Item 5. The composition of item 1 comprising:
   a) at least one oligoglycerol-containing lipid of Formula (I), 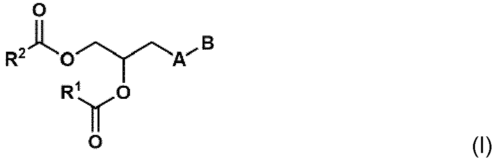
      wherein A = O-P(-O⁻)(=O)-O; or NR³ ; or N⁺R⁴R⁵ ;
      R¹ and R² each independently are a hydrocarbon group having 12 to 24 C-atoms;
      R³, R⁴ and R⁵ each independently are hydrogen or a hydrocarbon group having 1-5 C-atoms; and
      B = a linear or branched oligoglycerol group having from 2 to 20 glycerol units; and
   b)(iii) at least one helper lipid.
Item 6. The composition of any one of items 1 - 5, wherein the at least one oligoglycerol-containing lipid of Formula (I) comprises a phosphatidyloligoglycerol, wherein A = O-P(-O⁻)(=O)-O.
Item 7. The composition of any one of items 1 - 6, wherein the at least one oligoglycerol-containing lipid of Formula (I) comprises a cationic oligoglycerol containing lipid, wherein A = N⁺R⁴R⁵.
Item 8. The composition of any one of items 1 - 7, wherein the at least one oligoglycerol-containing lipid of Formula (I) comprises a cationizable oligoglycerol containing lipid, wherein A = NR³.
Item 9. The composition of any one of items 1 - 8, wherein the at least one oligoglycerol-containing lipid of Formula (I) comprises a phosphatidyloligoglycerol, wherein A = O-P(-O⁻)(=O)-O and a cationic oligoglycerol containing lipid, wherein A = N⁺R⁴R⁵.
Item 10. The composition of any one of items 1 - 9, wherein the at least one oligoglycerol-containing lipid of Formula (I) comprises from 2 to 4 glycerol units, preferably 2 or 3 glycerol units and more preferably 2 glycerol units.
Item 11. The composition of any one of items 1 - 10, wherein R¹ and R² independently are a linear or branched hydrocarbon group.
Item 12. The composition of any one of items 1 - 11, wherein R¹ and R² independently are a saturated or a monounsaturated or a polyunsaturated hydrocarbon group.
Item 13. The composition of any one of items 1 - 12, wherein R¹ and R² independently are a hydrocarbon group having 13 to 19 C-atoms and, in particular 15 or 17 C-atoms.
Item 14. The composition of any one of items 1 - 13, wherein R¹ and R² are a linear saturated C13- to C19- alkyl group.
Item 15. The composition of any one of items 1 - 14, wherein R³ = C₁-C₅-alkyl, in particular R³ = CH₃, and/or R⁴ and R⁵ each independently are C₁-C₅-alkyl, and in particular R⁴ = R⁵ = CH₃.
Item 16. The composition of any one of items 1-15, wherein the at least one oligoglycerol-containing lipid is of Formula (II), 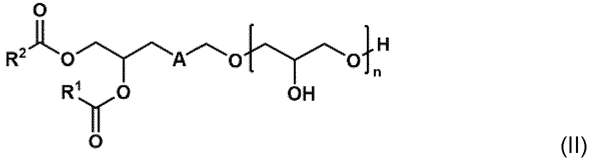 wherein = 2 to 20; and in particular n = 2 or 3.
Item 17. The composition of any one of items 1-16, comprising an oligoglycerol-containing lipid having the Formula 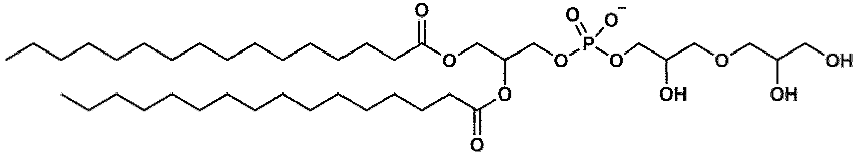
Item 18. The composition of any one of items 1-17, comprising an oligoglycerol-containing lipid having the Formula 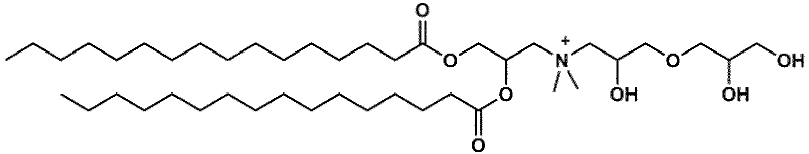
Item 19. The composition of any one of items 1-18, comprising an oligoglycerol-containing lipid having the Formula 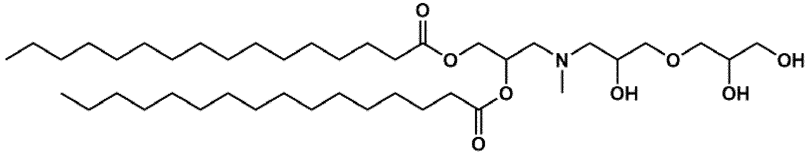
Item 20. The composition of any one of items 1 to 19, comprising,
   (a) at least one oligoglycerol-containing lipid of Formula (I), optionally
      (i) at least one phosphatidyloligoglycerol of Formula (I),
         wherein A = O-P(-O⁻)(=O)-O; and
      (ii) at least one cationic oligoglycerol-containing lipid of Formula (I),
         wherein A = N⁺R⁴R⁵; and
   (b)(i) at least one cationizable lipid; and
   (b)(ii) at least one structural lipid.
Item 21. The composition of any one of items 1 to 20, comprising
   (a) at least one oligoglycerol-containing lipid of Formula (I); and
   (b)(iii) at least one helper lipid, preferably at least two helper lipids.
Item 22. The composition of any one of items 1 - 21 being a lipid nanoparticle (LNP).
Item 23. The composition of any one of items 1-22 being a multi-layer lipid nanoparticle.
Item 24. The composition of any one of items 1 to 23, wherein the structural lipid is a sterol, preferably selected from the group consisting of cholesterol, fecosterol, sitosterol, ergosterol, campesterol, stigmasterol, brassicasterol, tomadidine, ursolic acid and α-tocopherol; and in particular wherein the structural lipid is cholesterol.
Item 25. The composition of any one of items 1-24, wherein the helper lipid is selected from the group consisting of
   hydrogenated soy L-α-phosphatidylcholine (HSPC);
   1,2-distearoyl-sn-glycero-3-phosphocholine (DSPC);
   1,2-diolioyl-sn-glycero-3-phosphoethanolamin (DOPE);
   1,2-dipalmitoyl-sn-glycero-3-phosphocholine (DPPC);
   1,2-dimyristoyl-sn-glycero-phosphocholine (DMPC);
   1,2-diolioyl-sn-glycero-3-phosphocholine (DOPC); and
   1-palmitoyl-2-olioyl-sn-glycero-3-phosphocholine (POPC);
   and in particular wherein the helper lipid is
   1,2-distearoyl-sn-glycero-3-phosphocholine (DSPC);
Item 26. The composition of any one of items 1, 2, 3 and 5-25, comprising b)(iv) a PEG lipid.
Item 27. The composition of any one of items 1-3 and 5-26, wherein the composition does not comprise a PEG lipid.
Item 28. The composition of any one of items 1, 2, 4, 6-20, 22-24 and 26-27 wherein the composition does not comprise a helper lipid.
Item 29. The composition of any one of items 1, 2, 4, 6-20, 22-24 and 27, wherein the composition does not comprise a helper lipid and does not comprise a PEG lipid.
Item 30. The composition of any one of items 1-29, further comprising
   c) a therapeutic and/or prophylactic agent.
Item 31. The composition of item 30, wherein the therapeutic agent is a nucleic acid, in particular a ribonucleic acid (RNA).
Item 32. The composition of item 31, wherein the RNA is selected from the group consisting of messenger RNA (mRNA), antisense RNA (aRNA), small interfering RNA (siRNA), CRISPR-RNA (crRNA), asymmetrical interfering RNA (aiRNA), micro-RNA (miRNA), Dicer-substrate RNA (dsRNA), small hairpin RNA (shRNA), and mixtures thereof.
Item 33. The composition of item 30, wherein the therapeutic and/or prophylactic agent is a small molecule.
Item 34. The composition of any one of items 1-31, comprising:
   a) at least one cationizable lipid;
   b) cholesterol;
   c) at least one of 1,2-dipalmitoyl-*sn*-glycero-3-phosphodiglycerol and 1,2-dipalmitoyl-*sn*-glycero-3-phosphotriglycerol;
   d) at least one of 1,2-distearoyl-*sn*-glycero-3-phosphocholine (DSPC) and CLG₂ according to claim 18; and
   e) optionally a therapeutic and/or prophylactic agent.
Item 35. The composition of item 34,
   wherein the composition does not contain a PEG lipid.
Item 36. A multi-layer LNP comprising at least an outer lipid shell comprised of a lipid bilayer and inner lipid phases comprising at least one, preferably at least 2, at least 3, at least 4, at least 5 concentrically arranged lipid bilayers, wherein preferably the concentrically arranged lipid bilayers further enclose lipid micelles, preferably inverted micelles and comprising any one of the compositions of items 1-35.
Item 37. The multi-layer LNP of item 36 comprising DPPG₂ and/or DPPG₃ in an amount of at least 2 mol%, more preferably at least 5 mol%.
Item 38. The multi-layer LNP of items 36-37 comprising a lipid mixture of DPPC/DSPC/DPPG₂, preferably in a molar range of about 40 to about 60 mol% DPPC, about 15 to about 25 mol% DSPC and about 20 to about 40 mol% DPPG₂.
Item 39. The multi-layer LNP of item 36 comprising a lipid mixture of NLG₂/cholesterol/DSPC/ DSPE-PEG in a molar range of about 35 mol% to about 55 mol% NLG₂, about 30 mol% to about 50 mol% cholesterol, about 5 mol% to about 15 mol% DSPC and about 0 mol% to about 6.5 mol% DSPE-PEG, more preferably about 42 mol% to about 50 mol% NLG₂, about 39 mol% to about 47 mol% cholesterol, about 8 mol% to about 12 mol% DSPC and about 1 mol% to about 2 mol% DSPE-PEG.
Item 40. A process for providing multi-layer LNPs, preferably multi-layer LNPs of any of the items 36 - 39 comprising a dual centrifugation (DC).
Item 41. The process for providing multi-layer LNP of item 40, wherein an aqueous composition comprising a total lipid concentration of at least about 50 % (w/v), preferably at least about 55 %, more preferably at least about 60 % (w/w) is subjected to dual centrifugation.
Item 42. The process for providing multi-layer LNP of any of items 40 or 41, comprising at least the following steps in the following sequence:
   Step 1 comprising providing a first composition comprising a lipid mixture in a first aqueous medium, and preferably beads;
   Step 2 comprising subjecting the first composition to dual centrifugation to obtain an intermediate composition;
   Step 3 comprising diluting the intermediate composition obtained in Step 2 with a dilution medium to obtain a diluted composition;
   Step 4 comprising subjecting the diluted composition obtained in Step 3 to a second dual centrifugation to obtain a composition comprising multi-layer LNPs.
Item 43. A multi-layer LNP obtained or obtainable by the process of any of the items 40 -42.

## Claims

1. A composition comprising:
a) at least one oligoglycerol-containing lipid of Formula (I), 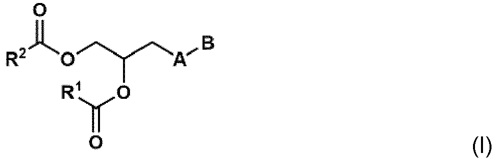
wherein A = O-P(-O⁻)(=O)-O; or NR³ ; or N⁺R⁴R⁵ ;
R¹ and R² each independently are a hydrocarbon group having 12 to 24 C-atoms;
R³, R⁴ and R⁵ each independently are hydrogen or a hydrocarbon group having 1-5 C-atoms; and
B = a linear or branched oligoglycerol group having from 2 to 20 glycerol units; and
b) at least one further lipid selected from
(i) at least one cationic or cationizable lipid;
(ii) at least one structural lipid;
(iii) at least one helper lipid; and/or
(iv) at least one PEG lipid.

2. The composition of claim 1 comprising:
a) at least one oligoglycerol-containing lipid of Formula (I), 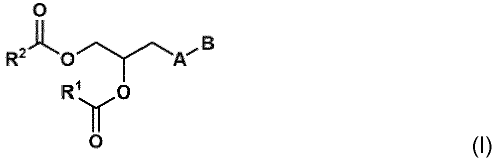
wherein A = O-P(-O⁻)(=O)-O; or NR³ ; or N⁺R⁴R⁵ ;
R¹ and R² each independently are a hydrocarbon group having 12 to 24 C-atoms;
R³, R⁴ and R⁵ each independently are hydrogen or a hydrocarbon group having 1-5 C-atoms; and
B = a linear or branched oligoglycerol group having from 2 to 20 glycerol units; and
b)(i) at least one cationic or cationizable lipid; and
b)(ii)at least one structural lipid.

3. The composition of claim 1 comprising:
a) at least one oligoglycerol-containing lipid of Formula (I), 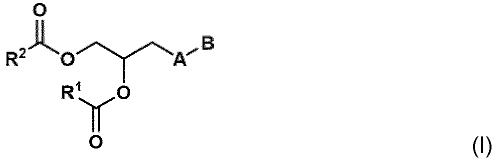
wherein A = O-P(-O⁻)(=O)-O; or NR³ ; or N⁺R⁴R⁵ ;
R¹ and R² each independently are a hydrocarbon group having 12 to 24 C-atoms;
R³, R⁴ and R⁵ each independently are hydrogen or a hydrocarbon group having 1-5 C-atoms; and
B = a linear or branched oligoglycerol group having from 2 to 20 glycerol units; and
b)(i) at least one cationic or cationizable lipid;
b)(ii)at least one structural lipid; and
b)(iii) at least one helper lipid.

4. The composition of claim 1 comprising:
a) at least one oligoglycerol-containing lipid of Formula (I), 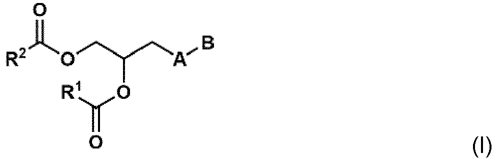
wherein A = O-P(-O⁻)(=O)-O; or NR³ ; or N⁺R⁴R⁵ ;
R¹ and R² each independently are a hydrocarbon group having 12 to 24 C-atoms;
R³, R⁴ and R⁵ each independently are hydrogen or a hydrocarbon group having 1-5 C-atoms; and
B = a linear or branched oligoglycerol group having from 2 to 20 glycerol units; and
b)(i) at least one cationic or cationizable lipid;
b)(ii)at least one structural lipid; and
b)(iv) at least one PEG-lipid.

5. The composition of claim 1 comprising:
a) at least one oligoglycerol-containing lipid of Formula (I), 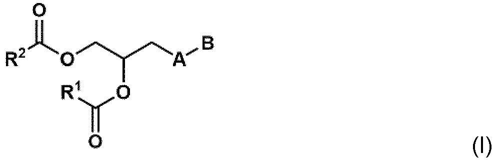
wherein A = O-P(-O⁻)(=O)-O; or NR³ ; or N⁺R⁴R⁵ ;
R¹ and R² each independently are a hydrocarbon group having 12 to 24 C-atoms;
R³, R⁴ and R⁵ each independently are hydrogen or a hydrocarbon group having 1-5 C-atoms; and
B = a linear or branched oligoglycerol group having from 2 to 20 glycerol units; and
b)(iii) at least one helper lipid.

6. The composition of any one of claims 1-5, comprising an oligoglycerol-containing lipid having the Formula 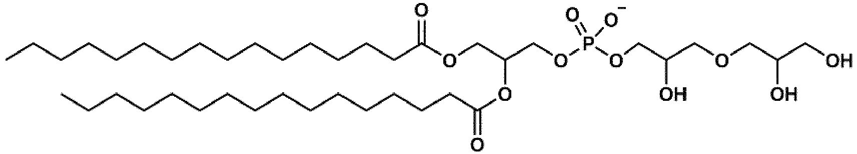 and/or comprising an oligoglycerol-containing lipid having the Formula 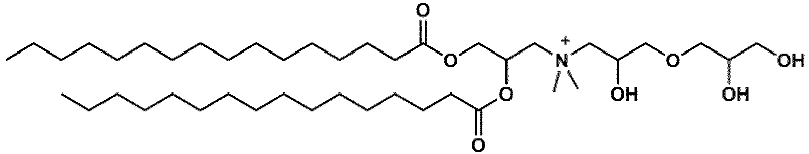 and/or comprising an oligoglycerol-containing lipid having the Formula

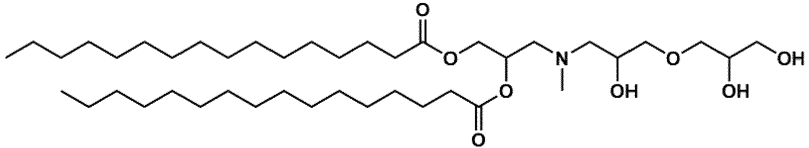

7. The composition of any one of claims 1 to 6, comprising,
(a) at least one oligoglycerol-containing lipid of Formula (I), optionally
(i) at least one phosphatidyloligoglycerol of Formula (I),
wherein A = O-P(-O⁻)(=O)-O; and
(ii) at least one cationic oligoglycerol-containing lipid of Formula (I),
wherein A = N⁺R⁴R⁵; and
(b)(i) at least one cationizable lipid; and
(b)(ii) at least one structural lipid.

8. The composition of any one of claims 1 to 7, comprising
(a) at least one oligoglycerol-containing lipid of Formula (I); and
(b)(iii) at least one helper lipid, preferably at least two helper lipids.

9. The composition of any one of claims 1 - 8 being a lipid nanoparticle (LNP).

10. The composition of any one of claims 1 - 9 being a multi-layer lipid nanoparticle.

11. The composition of any one of claims 1 to 10, wherein the structural lipid is a sterol, preferably selected from the group consisting of cholesterol, fecosterol, sitosterol, ergosterol, campesterol, stigmasterol, brassicasterol, tomadidine, ursolic acid and α-tocopherol; and in particular wherein the structural lipid is cholesterol.

12. The composition of any one of claims 1-11, wherein the helper lipid is selected from the group consisting of
hydrogenated soy L-α-phosphatidylcholine (HSPC);
1,2-distearoyl-*sn*-glycero-3-phosphocholine (DSPC);
1,2-diolioyl-*sn*-glycero-3-phosphoethanolamin (DOPE);
1,2-dipalmitoyl-*s*n-glycero-3-phosphocholine (DPPC);
1,2-dimyristoyl-*sn*-glycero-phosphocholine (DMPC);
1,2-diolioyl-*sn*-glycero-3-phosphocholine (DOPC); and
1-palmitoyl-2-olioyl-*sn*-glycero-3-phosphocholine (POPC);
and in particular wherein the helper lipid is
1,2-distearoyl-*sn*-glycero-3-phosphocholine (DSPC);

13. The composition of any one of claims 1-12, further comprising
c) a therapeutic and/or prophylactic agent.

14. The composition of claim 13, wherein the therapeutic agent is a nucleic acid or a small molecule, in particular a ribonucleic acid (RNA), preferably
selected from the group consisting of messenger RNA (mRNA), antisense RNA (aRNA), small interfering RNA (siRNA), CRISPR-RNA (crRNA), asymmetrical interfering RNA (aiRNA), micro-RNA (miRNA), Dicer-substrate RNA (dsRNA), small hairpin RNA (shRNA), and mixtures thereof.

15. The composition of any one of claims 1-14, comprising:
a) at least one cationizable lipid;
b) cholesterol;
c) at least one of 1,2-dipalmitoyl-*sn*-glycero-3-phosphodiglycerol and 1,2-dipalmitoyl-sn-glycero-3-phosphotriglycerol;
d) at least one of 1,2-distearoyl-*sn*-glycero-3-phosphocholine (DSPC) and CLG₂ according to claim 18; and
e) optionally a therapeutic and/or prophylactic agent.
